# EUROPEAN PATENT APPLICATION

(11) **EP 2 982 682 A1**
(43) Date of publication of application: **10.02.2016**
(21) Application number: 14778337.7
(22) Date of filing: 27.03.2014
(51) Int. Cl.: C07K 14/00, A61K 38/16, A61P 35/00

(54) **CHIMERIC PEPTIDE AND PHARMACEUTICAL COMPOSITION FOR TREATING ONCOLOGICAL DISEASES**

(30) Priority: 03.04.2013 EA 201300313
(71) Applicant: Obshestvo S Ogranichennoj Otvetstvennost`yu "Metamax", Moscow 109240 (RU)
(72) Inventor: BOZHENKO, Vladimir Konstantinovich, Moscow 121609 (RU)
(74) Representative: Jones Day
(86) International application number: PCT/RU2014/000199
(87) International publication number: WO 2014/163535

(57) **Abstract**

The invention relates to the field of biotechnology and medicine, and more particularly to novel sequence variants of a chimeric peptide comprising a functional fragment and a transporting sequence, wherein the functional fragment comprises an amino acid sequence selected from the group SEQ ID NO: 1 - SEQ ID NO: 17, as well as homologous sequences thereof with at least 60% sequence identity thereto; the invention further relates to a functional peptide with anti-proliferation activity represented by an amino acid sequence selected from the group SEQ ID NO: 1 - SEQ ID NO: 17, as well as homologous sequences thereof with at least 60% sequence identity thereto. The invention also relates to the use of the above-mentioned chimeric peptide for treating oncological diseases, as well as to a pharmaceutical composition having anti-proliferation activity and a method for treating oncological diseases, which comprises administration of said chimeric peptide to a mammal in need of such treatment. The invention makes it possible to develop a drug preparation which effectively penetrates target cells and has a high cytostatic and cytotoxic effect towards tumour cells.

## Description

### Technical Field Related to the Invention

The invention relates to the field of biotechnology and medicine, and more particularly to novel sequence variants of a chimeric peptide comprising a functional fragment and a transporting sequence, wherein the functional fragment comprises an amino acid sequence selected from the group SEQ ID NO: 1 - SEQ ID NO: 17, as well as homologous sequences thereof with at least 60% sequence identity thereto; the invention further relates to a functional peptide with antiproliferation activity represented by an amino acid sequence selected from the group SEQ ID NO: 1 - SEQ ID NO: 17, as well as homologous sequences thereof with at least 60% sequence identity thereto.

The invention also relates to the use of the above-mentioned chimeric peptide for treating oncologic diseases, as well as to a pharmaceutical composition having antiproliferation activity and a method for treating oncologic diseases, which comprises administration of said chimeric peptide to a mammal in need of such treatment. The invention makes it possible to develop a drug preparation, which effectively penetrates target cells and has a high cytostatic and cytotoxic effect towards tumour cells.

**Description of the Prior Art** Since the early 90-ies, more than 400 thousand cases of malignant neoplasms are diagnosed each year (M. I. Davydov, Y. M. Aksel, 2008). At the same time, in Europe the annual cancer mortality rate during the period from 1985 till 2002 continues to increase. The oncologic diseases occupy the second place next to the cardiovascular conditions in the structure of death causes. Therefore, the search for new anticancer drugs is one of the urgent objectives of the modem biology and medicine.

Currently, a lot of works are devoted to creation of new anticancer drugs based on the molecular biology achievements (Richard J.P. et al., 2003; Takeshima K. et al., 2003; Jyotika A. et al., 2005, B.P. Kopnin, 2000). It is generally accepted that a fundamental feature of a neoplastic cell is violation of the cell cycle regulation and apoptosis (Chappuis P.O., Kapusta L., 2005). It is known that regulation of the cell proliferation processes is controlled through sequential activation of cyclins and cyclin-dependent kinases (CDK). Cyclin kinases activity is determined by the expression level of the respective cyclins and the activity of specific cyclin kinases inhibitors (Kastan M.B., Bartek J., 2004). There are several families of cyclin-kinases inhibitors. The most studied and practically important are the following: p16INK4a, p21CIP/KIP, p27 KIP1 (Lowe S.W. et al., 2004). Mutations or promoters hypermethylation of cyclin kinases inhibitors genes are observed in 40-60% of malignant lymphomas, pancreatic cancer and several other malignant neoplasms (Sawyers C., 2004; Ortega S. et al., 2002).

Based on these results, several low molecular cyclin kinase inhibitors were synthesized. Another possible way to create cyclin kinase inhibitors can be presented by using the functional sequences and corresponding intracellular inhibitors (Ziegler A. et al., 2005). Pi6 INK4 protein is one of the most interesting candidates of the Cdk inhibitors group (Xu D. et al., 2004; Zhang Y. et al., 2005). It is well-known that p16INK4a protein inhibits cyclin D-dependent kinases, and, thus, the run of the G1 phase of the cell cycle (Fu G.H. et al., 2005; Ben-SaadonR. et al., 2004). It is showed that its function is impaired in a broad spectrum of oncologic diseases (Li J.Q. et al., 2004). Recently experimental works, describing gene p16INK4a application for gene therapy of tumours of different genesis, have appeared (Lee A.W.C., Li J-H et. al. 2003; Liu S.X., Tang S.Q., Liang C.Y., 2003; Zhang Y., Liu J. et al. 2005).

The additional motive to seek for application technologies related to natural protein proliferation inhibitors use was presented by the discovery of short amino acid sequences (n = 15-30), able to perform vector (transporting) functions with respect to peptide sequences and compounds of other chemical nature (RNA, DNA) (Fawell S., Seery J.et al., 1994; Vives E., Brodin P., Lebleu B. 1997; Kaplan I.M. et al., 2005; Gupta B. et al., 2005;Femandez-Cameado J. et al., 2005).

Up to the present moment the scientists tried to solve the problem of restoring the impaired function of intracellular proteins basing on gene delivery methods (gene therapy) (G. P. Georgiyev, 2000; Wender P.A. et al., 2000; A. Y. Baryshnikov, 2004). However this technology has not been widely applied in clinical practice yet due some essential problems. The alternative way to solve this problem, based on the technology of peptide vectors, which are capable to penetrate into cells without damaging the plasma membrane, is very promising due to the poor immunogenicity of such compounds and the ability to transfer fairly large molecules.

The combination of possibility of target peptide delivery to the cell and detection of short functional domains in protein regulators of different cellular functions has predetermined the construction of molecules with pathogenetic characteristics (Schutze-Redelmeier M.P. et al., 2004; Trehin R., Merkle H.P., 2004; Cong-Mei Wu et al., 2004). Relative simplicity of such molecules synthesis gives an opportunity to suggest an essential possibility of creation of new individual chemotherapy drugs based on them, i.e. the drugs influencing on the pathologic changes, typical for the given tumour (Perea S.E. et al., 2004).

The discovery of peptides, which are able to penetrate into the cell without membrane proteins participation as well as to perform the intracellular transportation of related protein fragments and oligonucleotides, opens a new stage in the development of biology and medicine. Peptide pAntp is one of the effective carriers of the large molecules, delivering them into cells. Its properties are well known in particular from the publications of Derossi D. et al. The third helix of the Antennapedia homeodamain translocates through membranes.// J.Biol. Chem. 269 (1994) 10444-10450 and Morris MC. et al. A peptides carrier for the delivery of biologically active proteins in mammalian cells.// Nat. Biotechnology. 19(2001) 1173-1176.

According to the author the closest equivalent of the present invention is the patent US 6569833 B1 (Cyclacel Limited, GB). This document describes the peptides, which bind with cyclin kinases and include amino acid residues 84-103 of complete-chain protein p16, and can be combined with the sequence of delivering protein, penetratin, by means of disulfide bond, appearing between cysteine residues, which are attached to C- terminus of peptide p16 and N- terminus of peptide Antp. The disadvantage of such method is the necessity of selective and multi-step synthesis of chimeric molecule, and that complicates the scheme of producing a target product and increases overall time required for synthesis.

### Summary of the Invention

This invention relates to biotechnology and medicine.

The object of this invention is to create a new chimeric peptide with increased therapeutic effect, which doesn't require a complicated and demanding scheme of production.

The technical result of the invention is an improved biomedical effect, objectively manifesting in prominent cytotoxic influence of the said chimeric peptide on tumour cells of such diseases as colorectal cancer, renal cancer, lung cancer, breast cancer, bladder cancer, pancreatic cancer, uterine cancer, prostate cancer, stomach cancer, ovarian cancer, myeloma, malignant lymphoma and breast fibroadenoma combined with other similar peptides, and additionally the simplicity of chimeric peptidase production due to reduced number of stages should be noted. The second technical result is presented by expanding the number of drugs for treatment of oncologic diseases. In addition, the author of the invention has suddenly found a prominent synergetic effect in case of combined use of the said chimeric peptide and already existing chemotherapy antitumour drugs (Etoposide, Taxol, 5-Fluororacil).

The technical result is achieved by obtaining a chimeric peptide with antiproliferative effect, containing a functional fragment and transport sequence, wherein the functional fragment includes an amino acid sequence SEQ ID NO: 1 or SEQ ID NO: 2, or SEQ ID NO: 3, or SEQ ID NO: 4 or SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15 or SEQ ID NO: 16 or SEQ ID NO: 17, and homologous thereto by 60% or more sequence.

Within one implementation of the invention it relates to the functional peptide with antiproliferative activity, including the amino acid sequence SEQ ID NO: 1 or SEQ ID NO: 2, or SEQ ID NO: 3, or SEQ ID NO: 4SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15 or SEQ ID NO: 16 or SEQ ID NO: 17, homologous thereto by 60% or more sequences.

Within other implementation of the invention it relates to the use of the said chimeric peptide for producing a drug indicated for treating oncologic diseases.

Within one preferable implementation of the invention it relates to the chimeric peptide, including the functional peptide, which is presented by the amino acid sequence SEQ ID NO: 1 or SEQ ID NO: 2, or SEQ ID NO: 3, or SEQ ID NO: 4.

Within other preferable implementation of the invention it relates to the functional peptide, presented by the amino acid sequence SEQ ID NO: 1 or SEQ ID NO: 2, or SEQ ID NO: 3, or SEQ ID NO: 4.

Within one preferable implementation of the invention it relates to the chimeric peptide, which can be used for treating a malignant tumour, chosen from the group, including colorectal cancer, renal cancer, lung cancer, breast cancer, bladder cancer, pancreatic cancer, uterine cancer, prostate cancer, stomach cancer, ovarian cancer, myeloma and malignant lymphoma.

Within the most preferable implementation the chimeric peptide can be used for treating colorectal cancer.

Within the preferable implementation the said drug can be used for treating a malignant tumour, chosen from the group, including colorectal cancer, renal cancer, lung cancer, breast cancer, bladder cancer, pancreatic cancer, uterine cancer, prostate cancer, stomach cancer, ovarian cancer, myeloma and malignant lymphoma.

Within the other implementation the invention relates to the pharmaceutical composition with antiproliferating activity, which includes the said chimeric peptide or functional peptide in combination with pharmaceutically acceptable carriers as an active substance.

Within the other implementation the invention relates to the pharmaceutical composition with antproliferating activity, which includes two active substances: one is the said chimeric peptide or functional peptide and the second - chemotherapy antitumour drug, chosen from the group, including alkylating agents, antimetabolites, plant alkaloids, antitumour antibiotics, platinum derivatives, camptothecin derivatives, altretamine, amsacrine, L-asparaginase, dacarbazine, estramustine, hydroxycarbamide, procarbazine, temozolomide, monoclonal antibodies, hormones, cytokines.

Within the preferable implementation the second active substance of the said pharmaceutical composition with antiproliferating activity can be a chemotherapy antitumour drug, chosen from the group, including Etoposide, Taxol and 5-Fluororacil.

Within other implementation the invention relates to the oncologic disease treatment scheme, which includes administration of the said chimeric peptide or pharmaceutical composition, or drug to the mammal in need of such treatment.

Within the preferable implementation of the abovementioned scheme the oncologic disease is chosen from the group, including colorectal cancer, renal cancer, lung cancer, breast cancer, bladder cancer, pancreatic cancer, uterine cancer, prostate cancer, stomach cancer and ovarian cancer.

Used herein, the term "chimeric peptide" includes a peptide with definite sequence of binding functional and delivery fragments in a chimeric molecule by sequence of any length, including additional amino acid residues (1 to 50), binding two said fragments, and concurrently the delivery amino acid sequence can be alternatively attached to the C-terminus of the said functional sequence through X group, where X is presented by the amino acid sequence, containing 1 to 50 amino acid residues. Methods of chimeric peptide production include solid-phase one, described in RU2297241, 22.11.2004 and RU2435783, 29.09.2010, as well as genetic engineering one, described in RU2297241, 22.11.2004 and RU2435783, 29.09.2010. Study of these chimeric peptides properties are described in RU2435783.

### The Structure of the Study Internalized Peptides

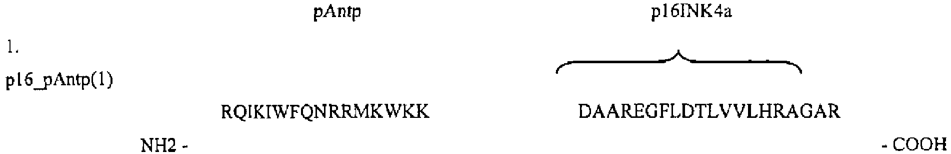

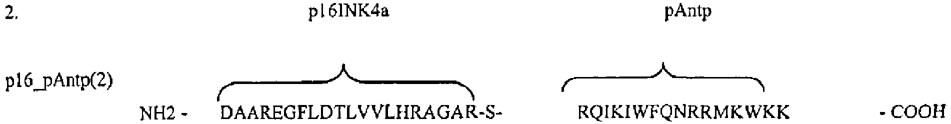

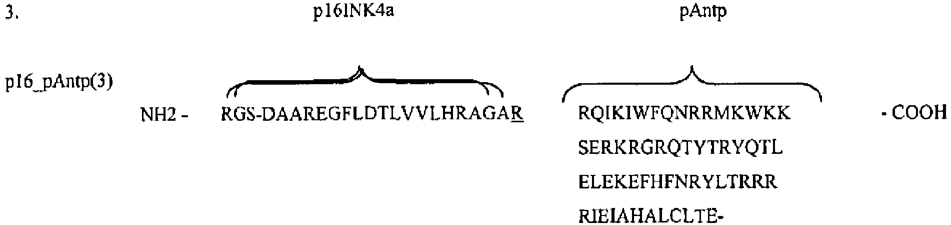

Peptides 1-3 consist of the functional part p16INK4a and internalized sequence pAntp, and differ in location of the functional group with respect to N-, C-terminus of the molecule as well as in the presence of insertion for peptide 3 (obtained with the help of genetic engineering).

Peptides p16_pAntp (1-2) differ in location of the functional group p16INK4a: peptide 1 - p16INK4a is located at the molecule C-terminus, peptide 2 - p16INK4a - at the N-terminus, the peptides are obtained with the help of solid-phase synthesis. In case of chimeric peptide p16_pAntp(3), obtained with the help of genetic engineering, the functional group p16INK4a is located at N-terminus, but there is an insertion from 44 AKO.

Used herein, the term "functional fragment" represents any polypeptide sequence of any size, or any structure, or any molecule, for the purpose of described functions performance, including amino acid sequence SEQ ID NO: 1 or SEQ ID NO: 2, or SEQ ID NO: 3, or SEQ ID NO: 4 SEQ ID NO: 5 or SEQ ID NO: 6 or SEQ ID NO: 7 or SEQ ID NO: 8 or SEQ ID NO: 9 or SEQ ID NO: 10 or SEQ ID NO: 11 or SEQ ID NO: 12 or SEQ ID NO: 13 or SEQ ID NO: 14 or SEQ ID NO: 15 or SEQ ID NO: 16 or SEQ ID NO: 17 as well as homologues thereto by 60% and more sequences.

Used herein, the term "antiproliferative activity" includes the ability of cyclin kinase inhibitors or compositions, including cyclin kinase inhibitors, to have a cytostatic and cytotoxic impact on malignant and benign tumour cells.

Used herein, the term "transport sequence" includes any transport sequence. Any transport sequence can be used, provided the claimed functional sequence, contained in any other polypeptide sequence of any size, or any other structure, or molecule, perform the functions described herein. The sequence of peptide VP22 (pAntp), herpes simplex virus, also can be used as delivery agent for carrying the cyclin kinase inhibitor into the target cells, for example, RQIKIWFQNRRMKWKK (SEQ ID NO: 6). The same is for peptide Tat sequence. Tat is a protein, performing a transactivation function in AIDS.

Used herein, the term "pharmaceutical composition" includes the composition containing a chimeric peptide or functional peptide, and also can contain a pharmaceutically acceptable carrier. For the purposes of oral administration the pharmaceutically acceptable carrier can include the binding substances, wetting agents, disintegrators, fillers, solubilizers, dispersants, stabilizers, suspending agents, colorants and fragrances. For injectable preparations, the pharmaceutically acceptable carrier may include buffer agents, preservatives, analgesics, solubilizers, isotonic agents and stabilizers. For topical preparations a pharmaceutically acceptable carrier may include bases, fillers, lubricants and preservatives. The pharmaceutical compositions of the present invention can be prepared in various drug forms using the aforementioned pharmaceutically acceptable carriers. For example, for oral administration, the pharmaceutical composition may be prepared in the form of tablets, pastilles, capsules, elixirs, suspensions, syrups or wafers. For drugs administered by injection, the pharmaceutical composition may be formulated in a single dose form, such as a vial, containing drugs for multiple administrations, or ampoules for single dose administration. The pharmaceutical composition may be formulated as solutions, suspensions, tablets, capsules, and sustained release formulations.

On the other hand, examples of the carriers, fillers and diluents suitable for producing the pharmaceutical preparations include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate and mineral oil.

Moreover, the pharmaceutical preparations may contain fillers, anticoagulants, lubricants, humectants, perfumes, emulsifiers and antiseptics.

In general the dose of the invented drug substance, which is used as a carrier, can be established depending on several factors, including type of the treating disease, administration type, age, gender and weight of the patient, disease severity, and type of the active drug substance.

Used herein, the term "alkylating agents" includes:
1. Alkyl sulfonates (Busulfan, Treosulfan).
2. Ethyleneimines (Thiotepa).
3. Nitrosourea derivatives (Carmustine, Lomustine, Mustophoran, Nimustin, Streptozocin),
4. Chloroethylamines (Bendamustin, Chlorambucil, Cyclophosphamide, Ifosfamide, Melphalan, Trofosfamide).

Used herein, the term "antimetabolites" includes:
1. Folic acid antagonists (Methotrexat, Ralitrexed).
2. Purine antagonists (Cladribin, Fludarabin, 6-Merkaptopurine, Pentostatin, Tioguanin).
3. Pyrimidine antagonists (Cytarabine, 5-Fluororacil, Capecitabine, Gemcitabine).

Used herein, the term "plant alkaloids" includes:
1. Podophyllotoxins (Etoposide, Teniposide).
2. Taxanes (Docetaxel, Paclitaxel).
3. Vinca alkaloids (*Vincristine*, Vinblastin, Vindesin, Vinorelbin).

Used herein, the term "antitumour antibiotics" includes:
1. Anthracycline (Daunorubicin, Doxorubicin, Epirubicin, Idarubicin, Mitoxantron).
2. Other antitumour antibiotics (Bleomicin, Dactinomycin, Mitomycin, Plicamycin).

Used herein, the term "platinum derivatives" includes: Carboplatin, Cysplatin, Oxaliplatin. Used herein, the term "camptothecin derivatives" includes: Irinotecan, Topotecan.

Used herein, the term "monoclonal antibodies" includes: Ederkolomab, Rituximab, Trastuzumab.

Used herein, the term "hormones" includes:
1. Antiandrogens (Bicalutamide, Cyproterone acetate, Flutamide).
2. Antiestrogens (Tamoxifen, Toremifene, Droloxiphen).
3. Aromatase inhibitors (Formestane, Anastrozole, Exemestane).
4. Progestins (Medroxyprogesterone acetate, Megestrol acetate).
5. LH-RH agonists (Buserelin, Goserelin, Leuprolin acetate, Triptorelin).
6. Estrogens (Phosphoestrol, Polyestradiol).

Used herein, the term "cytokines" includes:
1. Growth factors (Filgrastim, Lenograstim, Molgramostim, Erythropoetin, Trombopoietin).
2. Interferons (a-interferons, p-interferons, y-interferons).
3. Interleukins (interleukin-2, interleukin-3, interleukin-Π).

Used herein, the term "oncologic disease" includes malignant and benign tumours.

### Brief Description of Drawings

Fig. 1. Changes in the apoptosis level (Annexin-positive particles) of the blood mononuclears upon injecting in cellular medium of peptides D37K, F26K-1 and W26K-1 in different concentrations, average values. Incubation time 24 hours.
Fig. 2. Changes in the number of "live"/intact cells upon incubation of the blood mononuclear fraction cells of healthy donors with study peptide sequences D37K, F26K-1 and W26K-1 in different concentrations. Incubation time 24 hours.
Fig. 3. Changes in the apoptosis level of the cell culture HCT-116 upon incubation with the study peptide sequences. Incubation time 24 hours.
Fig. 4. Changes in the apoptosis level of the cell culture MCF-7 upon incubation with the study peptide sequences. Incubation time 24 hours.
Fig. 5. Changes in the number of dead cells, including propidium iodide, culture HCT-116, incubation with the study peptide sequences - 24 hours. Peptide concentration - 5 µM, 10 µM, 20 µM, 40 µM.
Fig. 6. Changes in the number of dead cells, including propidium iodide, culture MCF-7, incubation with the study peptide sequences - 24 hours. Peptide concentration - 5 µM, 10 µM, 20 µM, 40 µM.
Fig. 7. Changes in relative number of live cells in the culture HCT-116 upon incubation with the study peptide sequences during 24 hours. The number of live cells in the reference sample is taken as 100% (20 µM DMSO); concentrations of the study peptides comprised 5 µM, 10 µM, 20 M µM, 40 µM.
Fig. 8. Changes in number of live cells in the culture MCF-7 upon incubation with the study peptide sequences during 24 hours. Concentrations of the study peptides comprised 5 µM, 10 µM, 20 M µM, 40 µM.
Fig. 9. Changes in number of cells within phases S and G2/M upon 24-hour incubation of active proliferating cells of lines MCF-7 and HCT-116 with peptide sequences D37K, F26K-1, F26K-2, W26K-1, W26K-2 in concentrations of 10 and 40 µM.
Fig. 10. Changes in cells level in G0/G1-phase of the cell cycle in the culture HCT-116 upon incubation of the synchronized culture with the study peptide sequences in concentrations of 40 µM.
Fig. 11. Changes in cells level in G0/G1-phase of the cell cycle in the culture MCF-7 upon incubation of the synchronized culture with the study peptide sequences in concentrations of 40 µM.
Fig. 12. Changes in cells level in G0/G1-phase of the cell cycle in the culture A549 upon incubation of the synchronized culture with the study peptide sequences in concentrations of 40 µM.
Fig. 13. Changes in cells level in S-phase of the cell cycle in the culture A549 upon incubation of the synchronized culture with the study peptide sequences in concentrations of 40 µM.
Fig. 14. Differences in the level of S-phase in the cell culture A549, upon incubation during 0, 4, 6 and 8 hours with the peptide sequences F26K-1, W26K-1 and D37K in concentration of 40 µM.
Fig. 15. Changes in cells level in G0/G1-phase of the cell cycle upon incubation of the SKOV line cells with the study peptide sequences during 24 hours.
Fig. 16. Changes in number of the cells in phases S+G2/M of the cell cycle upon incubation of the SKOV line cells with the study peptide sequences during 24 hours.
Fig. 17. Changes in the apoptosis level of the cell culture SKOV upon incubation with the study peptides during 24 hours, estimated as hypodiploid peak on the DNA-histograms.
Peptide concentrations comprised 5 µM, 10 µM, 20 µM, 30 µM, 40 µM. The culture was preliminary synchronized in G0/G1-phase by means of depleted culture medium.
Fig. 18. Changes in the level of protein Bcl-2 in HCT-116 line cells upon incubation with the sequences D37K, F26K-1, F26K-2, W26K-1 and W26K-2. Incubation time 24 hours. After studying the changes in Bcl-2 level it was found that the peptides D37K and W26K-1 had minimal impact, decreasing the level of Bcl-2-positive cells in the culture by 5% upon incubation during 24 hours and peptide concentration of 40 µM. Peptides W26K-2 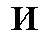 F26K-1 decreased the Bcl-2 level by more than 15%.
Fig. 19. Changes in the level of phosphorylated pRb in the culture MCF-7 upon incubation with the study peptide sequences D37K, F26K-1, F26K-2, W26K-1 and W26K-2. Incubation time 24 hours, peptide concentration: A - 10 µM, B - 40 µM.
Fig. 20. Changes in the level of "under-phosphorylated" pRb in the cell culture HCT-116 upon incubation with the study peptide sequences. A - peptide concentration - 10 µM, B - peptide concentration - 40 µM.
Fig. 21. Changes in the level of "under-phosphorylated" pRb in the cell culture SKOV upon incubation with the study peptide sequences. A - peptide concentration - 10 µM, B - peptide concentration - 40 µM.
Fig. 22. Dependence of the HCT-116 dead cells number from drugs concentration (Etoposide, Taxol). Cytotoxic effect was registered with the help of MTT-assay. Incubation time was 24 hours, the drugs concentrations - 1, 10 and 30 µM.
Fig. 23. Dependence of the HCT-116 dead cells number from the concentration of internalized sequences (F26K-1, D37K). Cytotoxic effect was registered with the help of MTT-assay. Incubation time was 24 hours, sequences concentrations - 1, 10 and 30 µM.
Cytotoxic effect appeared to be more significant for peptide F26K-1, there is concentration dependence.
Fig. 24. Combined influence on the HCT-116 cells of the sequence D37K and drugs (Taxol, Etiposide), concentrations - 1, 10 and 30 µM, incubation time - 24 hours.
Fig. 25. Combined influence on the HCT-116 culture of the sequence F26K-1 in concentrations of 1, 10 and 30 µM, as well as Etoposide in the same concentrations.
Incubation time - 24 hours, method - MTT-assay.
Fig. 26. Combined influence on the HCT-116 culture of the sequence F26K-1 in concentrations of 1, 10 and 30 µM, as well as Taxol in the same concentrations. Incubation time - 24 hours, method - MTT-assay.
Fig. 27. Study of the combined influence of Taxol and the sequences D37K (A) and F26K-2 (B) on the MCF-7. Preparations concentrations - 10, 20, 30 µM, incubation time - 24 hours.
Fig. 28. Study of the combined influence of 5-Fluorouracil and the sequences D37K (A) and F26K-2 (B) on MCF-7. Preparations concentrations 10, 20, 30 µM, incubation time - 24 hours.
Fig. 29. Changes in apoptosis level in H460 culture upon cells incubation with the peptide sequences (A), and combined influence of Etoposide and the sequences (B). Incubation time - 24 hours, PI staining, fixed samples.
Fig. 30. Changes in apoptosis level in H460 culture upon cells incubation with the peptide sequences (A), and combined influence of Etoposide and the sequences (B). Incubation time - 24 hours, AnnexinV-PI staining.
Fig. 31. Changes in apoptosis level in H460 culture upon cells incubation with the peptide sequences and preliminary incubation with Etoposide in concentration of 10 µM. Time of incubation with peptides - 24 hours, AnnexinV-PI staining.
Fig. 32. Changes in apoptosis level in H460 culture upon cells incubation with the peptide sequences and preliminary incubation with Etoposide in concentration of 10 µM. Time of incubation with peptides - 24 hours, PI staining with preliminary fixation of samples.
Fig. 33. Distribution of protein pAntp_p16 in cells A549 in the course of time. In 1, 15 and 30 min after protein addition. Leica Laser scanning microscope.
Fig. 34. Changes in the inoculated cells tumour volume of the thymus-deprived mice upon injection of the chimeric internalized peptide Antp_p16 into the tumour. The mice were inoculated with the cell culture A549 and injected the peptide Antp_p16 in doses of 0.1 and 0.2 mg.
Fig. 35. Changes in the inoculated cells tumour volume of the thymus-deprived mice upon injection of the chimeric internalized peptide Antp_p16 into the tumour. The mice were inoculated with HCT-116 cells, the peptide was injected in dose of 0.1 mg.

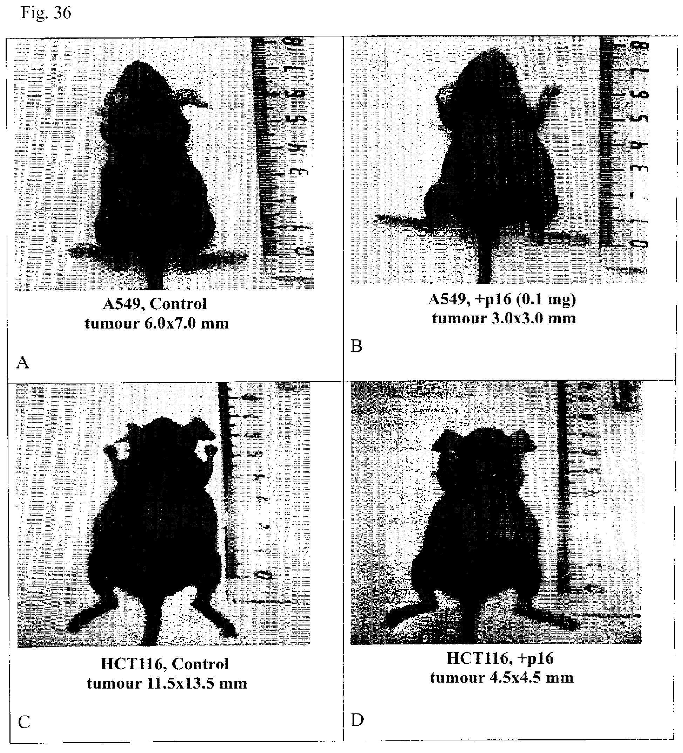

Fig. 36. Photos of test animals at the moment of 7^{th} injection of studied internalized peptide P16_Antp. A and B - photos of mice with transplanted cells A549, A-mouse from the control group on the day 16 of the test, the tumour is sized 6.0x7.0 mm. B-mouse from the test group on day 16 of test injected with 0.1 mg P16_Antp, tumour sized 3.0x3.0 mm. Figures C and D - mice with transplanted cells HCT-116, C - mouse from the control group on day 18 after tumour cells transplant (tumour sized 11.5x13.5 mm). D - mouse from the test group after 7 injections of 0.1 mg P16_Antp, tumour sized 4.5x4.5 mm.
Fig. 37. Two-parameter cytogram of breast cells. X-axis - PI staining, Y-axis - cytokeratine staining. "Cytokeratine-positive apoptosis" region is indicated with an arrow.

### Embodiment of the Invention

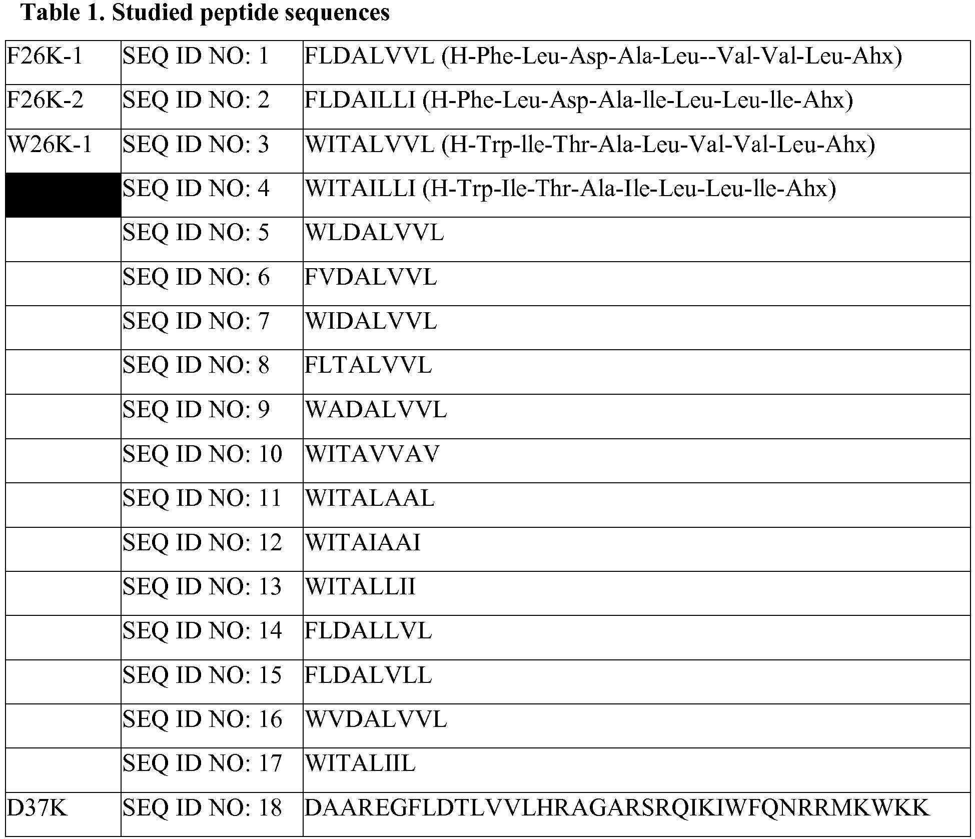

### Example 1. Study of effect of peptide sequences D37K, F26K-1, and W26K-1 on non-dividing mononuclear of peripheral blood

A number of tests were performed on mononuclear fraction of leucocytes of healthy donors in order to evaluate the cytotoxic effect on non-dividing blood cells for sequences D37K, F26K-1 and W26K-1. For this purpose we separated the mononuclear fraction from the whole venous blood with anticoagulant (EDTA) with density gradient p = 1.007, the cells were washed in PBS and placed into 24-wells plate in amount 150,000 per ml. To the cells suspension we added the study peptides in concentrations 10, 20, 30 40, 50 and 60 µM, peptides were dissolved in PBS. Incubation lasted for 24 hours at 37°C and 5% CO₂ in culture medium RPMI (10% PBS).

After incubation the cells were sampled into microvials, the medium was washed and the samples were stained using antibodies Annexin V (Recombinant human Annexin V FITC conjugate, CALTAG Laboratories, U.S.A.) and PI using manufacturer's method. The results were evaluated using Becman Coulter flow cytometer.

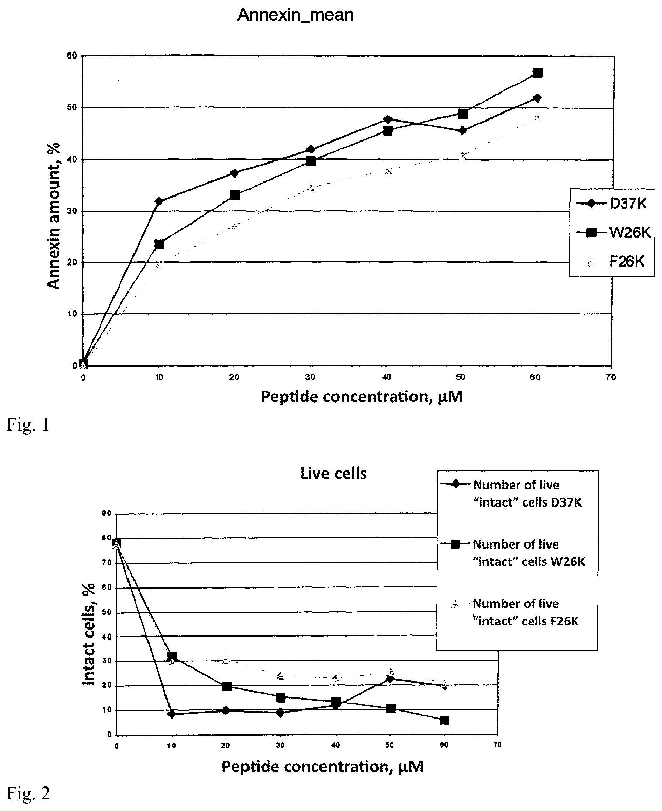

It was shown that the studied peptide sequences are capable of inducing apoptosis (Figure 1); there is clearly expressed concentration dependence of the apoptosis level of blood mononuclears from the amount of injected peptide, apoptosis values being close for 3 studied sequences. Amount of live cells, evaluated as a number of intact/non-stained cells during staining with Annexin V/ PI, abruptly decreases during the incubation with the studied peptides, which signifies expressed cytotoxic activity (Figure 2). At the same time the effect is more pronounced for the sequence D37K.

### Example 2. Study of the cytotoxic activity on the proliferating cell lines

We studied the effect of peptide sequences D37K, F26K-1, F26K-2, W26K-1, W26K-2 on the cells of transplanted cell cultures MCF-7 (breast cancer), A549 (lung adenocarcinoma), SKOV (ovarian cancer) and HCT116 (colon adenocarcinoma).

When preparing for the experiment the cells cultures were placed in 24-well plates in amount of 1 * 10⁴ cells per well, incubated for 48 hours at 37°C and 5% CO₂, medium DMEM (PanEco, Russia), containing glutamine, gentamicin, 10% PBS. After such incubation the cell cultures formed loose mono-layer. Experimental set-up: culture medium was replaced with the medium containing the study peptides in the concentrations of 5 µM, 10 µM, 20 µM, 40 µM; in the control samples the medium contained DMSO 20 µL to determine the effect of solvent (peptides were dissolved in DMSO). Incubation time was 24 hours. Then the cells were carefully removed from the matrix using trypsin (cells in the wells with peptides were attached stronger than the control sample), the medium was removed via centrifugation at 3,000 rounds for 7 minutes, supernatant fluid was removed and the staining was performed using double mark Annexin V/PI. Analysis was performed using flow cytometer and evaluated the number of cells including Annexin V - apoptosis, the number of cells including propidium iodide - dead cells, the number of intact cells - live cells.

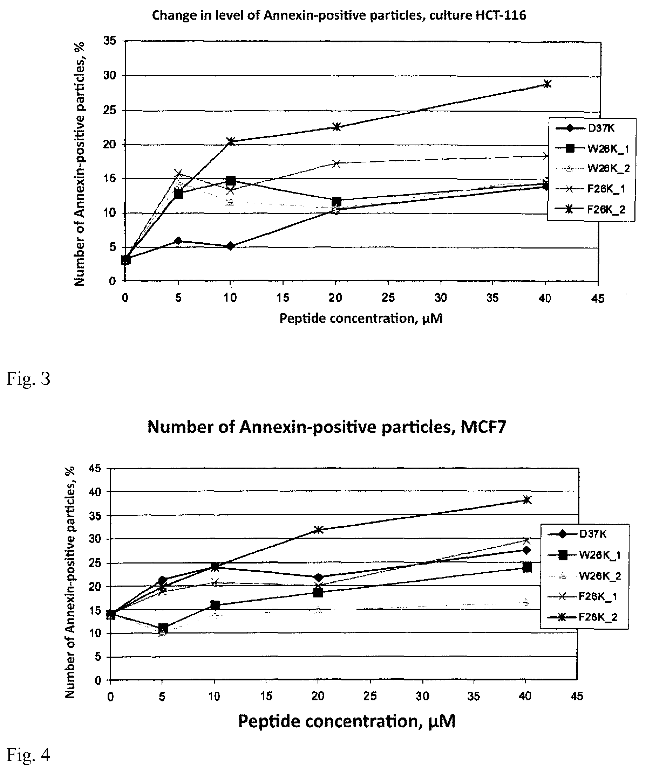

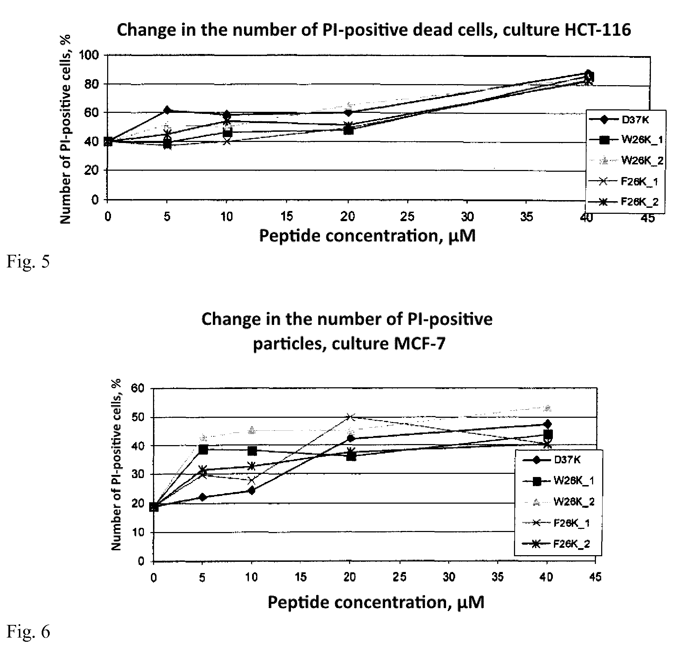

When studying the apoptosis level it was shown that injection of the peptide sequences into the culture medium of proliferating cells leads to increase of the apoptosis in the culture (figures 3,4), with apoptosis increasing proportionally to peptide concentration increase. The results showed that the peptide sequence F26K-2 (H-Phe-Leu-Asp-Ala-Ile-Leu-Leu-Ile-Ahx) has more pronounced pro-apoptosis effect on the studied cell lines. Regarding other studied sequences there was no significant differences in the level of induced apoptosis. Number of dead cells, including propidium iodide, also increases when adding peptides to culture medium and is concentration-dependent (Figures 5, 6).

When studying the amount of dead cells we failed to get significant differences regarding the degree of the peptides effect, however the number of dead cells in culture HCT-116 is higher than in the culture MCF-7, which is likely explained by higher level of spontaneous cells death in culture HCT-116.

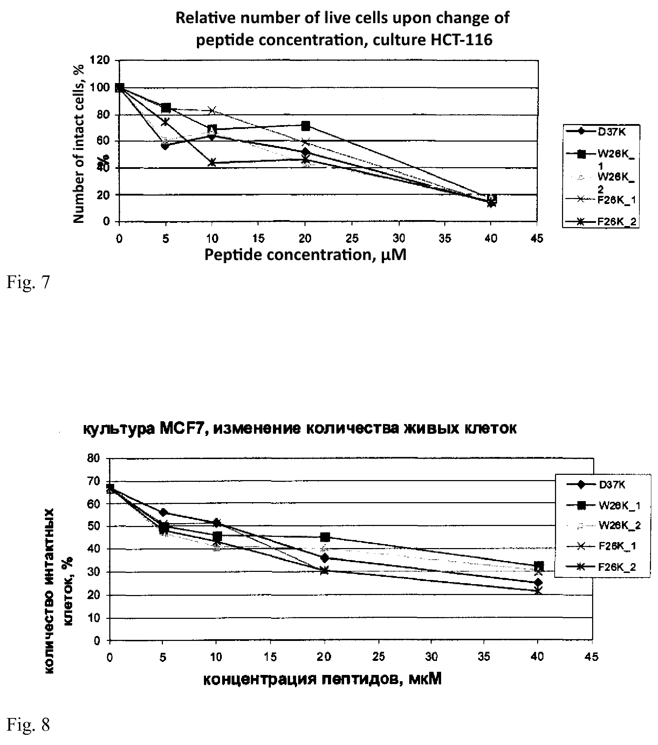

Number of live cells inversely correlates with the amount of the peptide sequences put into medium (Figures 7, 8).

When studying cytotoxic effect of the peptide sequences on cultures A549 and SKOV, we learned that the sequences F26K-1 and W26K-1 have the most potent effect, i.e. for culture A549 at sequences concentration of 40 µM the level of Annexin-positive cells was 43.5 and 52.1%, for sequences F26K-1 and W26K-1; the number of particles with subdiploid/fragmented DNA was 46.4 and 48.2%. When incubating with the studied sequences for 24 hours. When incubating the cells of line A549 with sequence D37K at concentration of 40 µM for 24 hours the number of Annexin-positive cells was 33.8%; the number of particles with fragmented DNA was 36.3%. In the control samples the amount of Annexin-positive particles was no more than 8%, the level of particles with fragmented DNA was no more than 10%.

For SKOV culture the values in the "early" apoptosis level (Annexin-positive particles) were 41.8% for the sequence F26K-1 and 45.2% for the sequence W26K-1. For the peptide sequence D37K the "early" apoptosis level was 37.6%. The number of particles with fragmented DNA in this culture with incubation for 24 hours with the sequences F26K-1 and W26K-1 was 42.6% and 44.1% respectively. The level of fragmented DNA in incubation with D37K sequence was 31.5%; the level of fragmented DNA in the control samples for both cultures was 8%.

There were no differences in changes of the number of live cells for different sequences.

### Example 3.Study of the cytostatic effect

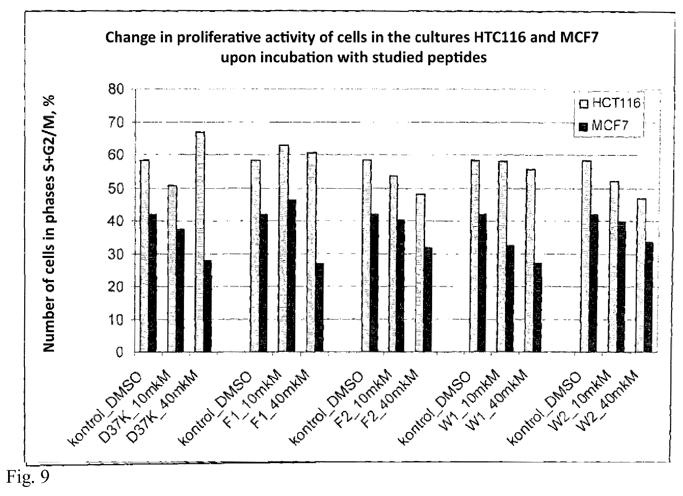

Using cell lines MCF-7, HCT-116, A549 (lung adenocarcinoma) and SKOV (ovarian cancer) we studied the effect of the peptide sequences on the cells proliferative activity. The experiments involved synchronization of the cells in G0/G1-phase of the cells cycle using depleted culture medium. The cells in 24-well plate were held for 48 hours in the medium containing 0.5% PBS, then the synchronization block was removed via replacement of the medium with one containing 5% PBS and the study peptides in different concentrations. It was incubated for 24 hours, and then the cells were removed from the matrix using trypsin, washed in PSB, fixed in ethanol. Staining of samples, washing from ethanol in PBS using centrifugation, incubation with RNA-ase, staining with propidium iodide. Analysis was performed using flow cytometry, we evaluated the distribution of cells in the cell cycle phases on DNA-histograms using ModFit 3.0 software. For cells cultures MCF-7 and HCT-116 we studied the effect of 5 peptide sequences in concentrations of 10 and 40 µM. It was shown that the study sequences are able to decrease the proliferating activity of the dividing cells (Figure 9).

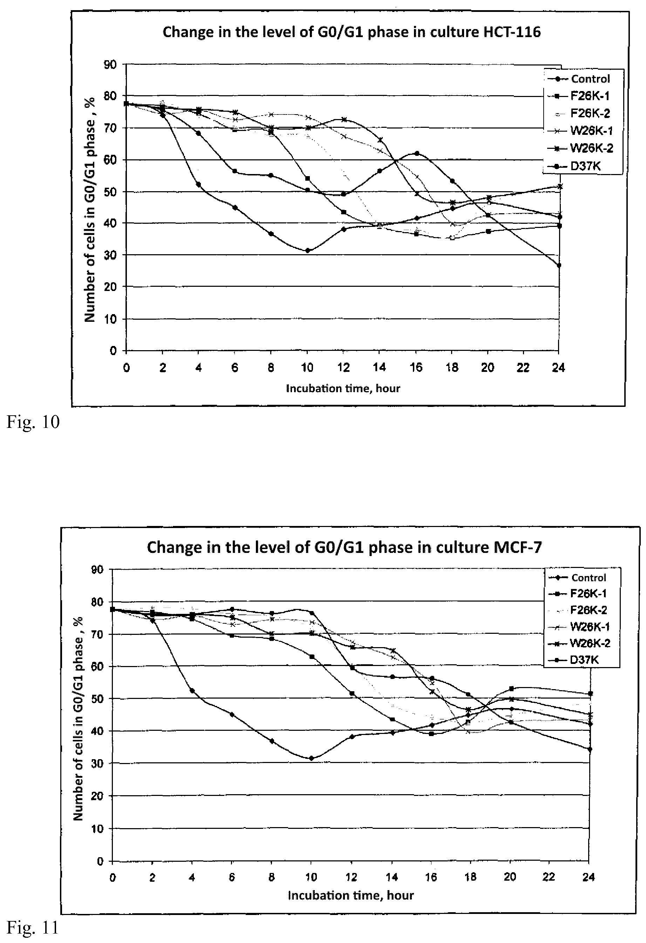

When studying the changes in distribution of cells in the cell cycle phases depending on incubation time with the sequences it was shown that the most clear antiproliferative effect and its concentration dependence are seen for sequences F26K-2, W26K-1, W26K-2. For sequence D37K antiproliferative effect is expressed for the cell line MCF-7, however, it can't be seen for the cell line HCT-116. Thus, for culture HCT-116 a delay in cells leaving the rest phase is 8-12 hours when exposed to the sequences F26K-1, F26K-2, W26K-1, W26K-2, the greatest delay was seen when incubating the cells with the sequence W26K-2 (12 hours, the number of cells in G0/G1-phase - 72%) (Figure 10).

For the culture MCF-7 a delay in proliferation (exit of cells after synchronization block) was seen for all studied peptide sequences, and there were no significant differences found between effects of sequences F26K-1, F26K-2, W26K-1, W26K-2 and D37K.

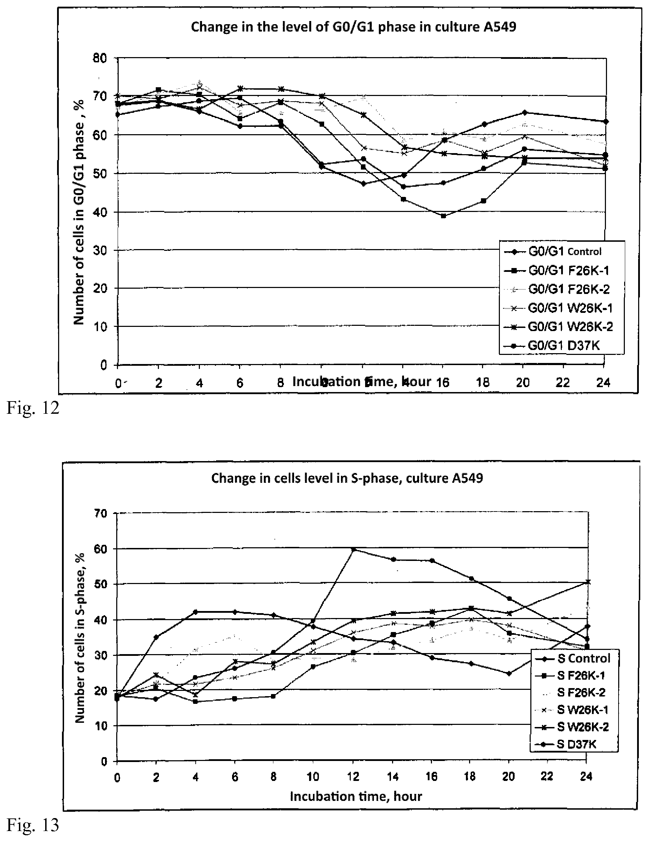

When estimating the changes in the levels of the cell cycle phase in the cell culture line A549, there was no delay in the exit of cells from phase G0/G1 (Figure 12).

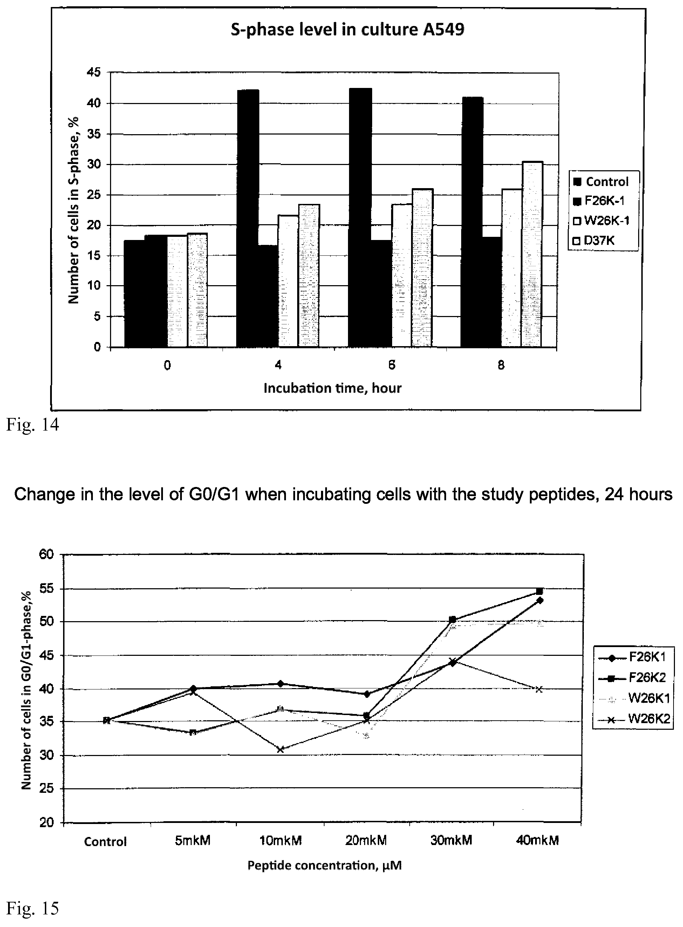

A549 line displayed more smooth exit from synchronization that the cultures MCF-7 and HCT-116, the cells of the control samples for up to 8 hours after incubation remained in phase G0/G1 in amount over 60%. The effect of the study sequences on the level of phase G0/G1 was minimal. However, when analyzing the changes in S-phase level we found that addition to the culture medium of such sequences as F26K-1, W26K-1 and D37K led to the delay of cells "entry" in S-phase. If in the control samples the S-phase level began to increase immediately after removal of the synchronization block, in the samples with sequences F26K-1, W26K-1 and D37K, S-phase level began to increase only 8 hours after the exclusion of a synchronizing factor (Figure 13, 14).

Thus for A549 cell culture line we got the delay at the second restriction point level, transition S - G2, for the sequences F26K-1, W26K-1 and D37K, which is probably explained by the peculiarities of the cell line.

When studying the effect of the peptide sequences on SKOV cells culture the incubation was performed with the sequences F26K-1, F26K-2, W26K-1, and W26K-2. The cells were pre-synchronized using depleted medium containing 0.5% PBS (fetal bovine serum), the peptides were entered into the culture medium together with removal of the synchronization block and the replacement of nutrition medium with the medium containing 10% PBS.

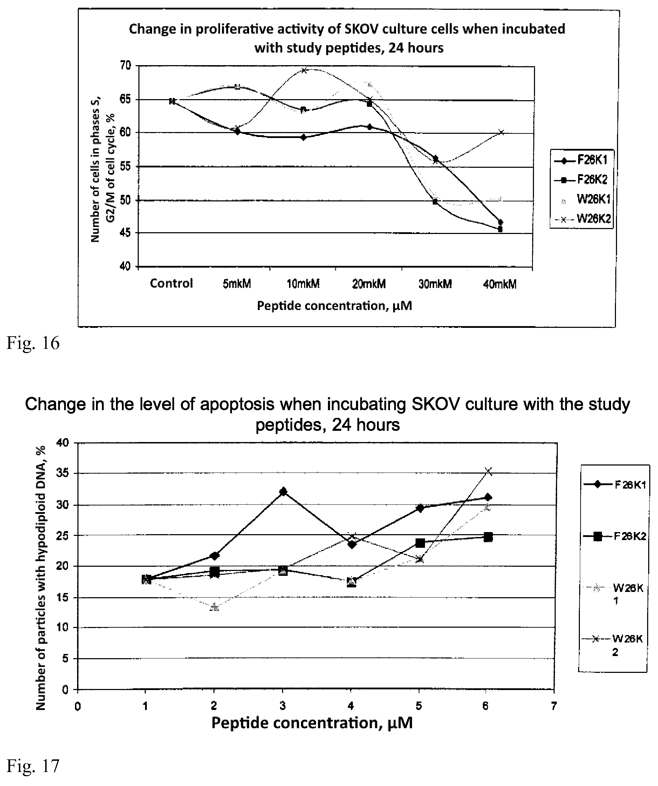

Figures 15, 16 show change in the number of cells in phases G0/G1 and S+G2/M, all the studied peptides display increase of the number of cells in G0/G1-phase and decrease of the proliferating cells (S+G2/M - phases) upon increasing a peptide concentration. Slightly lesser antiproliferative effect was observed in this case for the sequence W26K-2. The sequences F26K-1, F26K-2, W26K-1 caused the delay of proliferation of SKOV cell line without significant difference.

The apoptosis level estimated as a hypodiploid peak on DNA-histograms, correlates with the concentration of the injected peptide and increases from 17.8% in the control sample to 31.2% in 24 hours incubation with the peptide F26K-1 in concentration of 40 µM, and to 35.3% in incubation with the peptide W26K-2 (40 µM) (Figure 17).

When attempting to test the cytostatic effect of the peptide sequences using other methods of synchronization: using hydroxyurea - stop in G0/G1-phase, Taxol - stop in G2-phase and Eptoposide -stop in S-phase, no reliable results were received. The test data when adding the studied sequences to the cell cultures displayed cells death for over 70%, excluding the possibility of an adequate analysis of cells distribution in the cell cycle phases.

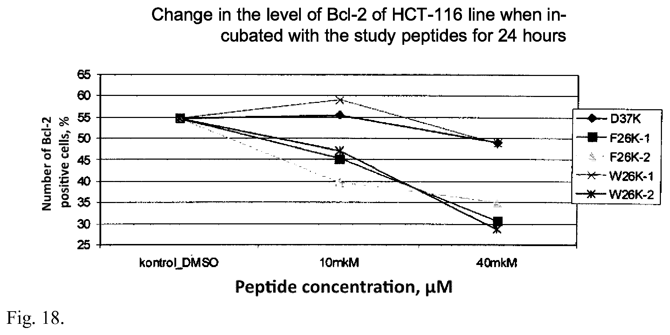

***Example 4. Investigation of the peptide sequences effect on change in the level of Bcl***-***2*** Monoclonal antibodies Anti Human Bcl-2 (Caltag Laboratories, USA) were used to estimated change in the level of Bcl-2 in HCT-116 cells when incubated with the study peptide sequence. The cells were transferred to a 24-well plate at 1 x 10 4 per well, grown for 48 hours under normal conditions (medium DMEM, 10%), then synchronized using the depleted medium containing 0.5% PBS for 48 hours. Upon removing the synchronizing block the medium containing 10% PBS was added to the cells, and the peptide sequences were studied at concentrations of 10 and 40 µM. Incubation with the peptides was performed for 24 hours, then the culture was removed with the help of trypsin, fixed for one hour with PBS containing 1% formaldehyde, and then fixed with ethanol. The staining and the analysis were performed according to the antibodies manufacturer's instructions. It was found that the investigated sequences are capable of inhibiting the expression of Bcl-2protein, the strongest inhibition was influenced by the sequences F26K-1, F26K-2, W26K-2, and for those sequences we also got the dependence level of Bc2-2 on peptide concentration (Figure 18). It is thereby shown that the apoptosis induced by introduction of the peptide sequences into the culture medium is explained by influence of the peptides on specific molecular processes of the cell.

In the study of changes in the Bc2-2 level it was found that the peptides D37K and W26K show minimal effect, reducing the level of Bcl-2-positive cells in the culture by 5% at 24 hours of incubation and the peptide concentration of 40 µM. Peptides W26K and F26K-2 decreased the level of Bcl-2 by over 15%.

### Example 5. Investigation of changes in the amount of phosphorylated pRb during the incubation of cell cultures with the peptide sequences

One of the objectives of this work was to prove that the antiproliferative effects of the study peptides are associated with a specific action of the cyclin kinase inhibitor fragments within their structure. Although the literature describes the retention of such peptides properties to inhibit a phosphorylating function for cyclin-dependent kinases, this was confirmed in cell extracts. We monitored changes in the level of pRB phosphorylation - molecular target of type D cyclin kinase against the background of the investigated peptides, including the sequences, having homology with p16INK4a, at the cellular level with the help of flow cytometry method.

Activation of the own cell p16INK4a leads to inhibition of pRb phosphorylation. Accumulation of the underphosphorylated pRb leads to inhibition of E2F and reduction of cyclins A and B expression, which is one of the key mechanisms in the cell cycle arrest. By the amount of phosphorylated pRb, one can estimate the activity of ρ16.

Flow cytometry allows to render the cells, where the pRb product is in the "underphosphorylated" state. The fluorescently labeled antibodies interact with "underphosphorylated" pRb.

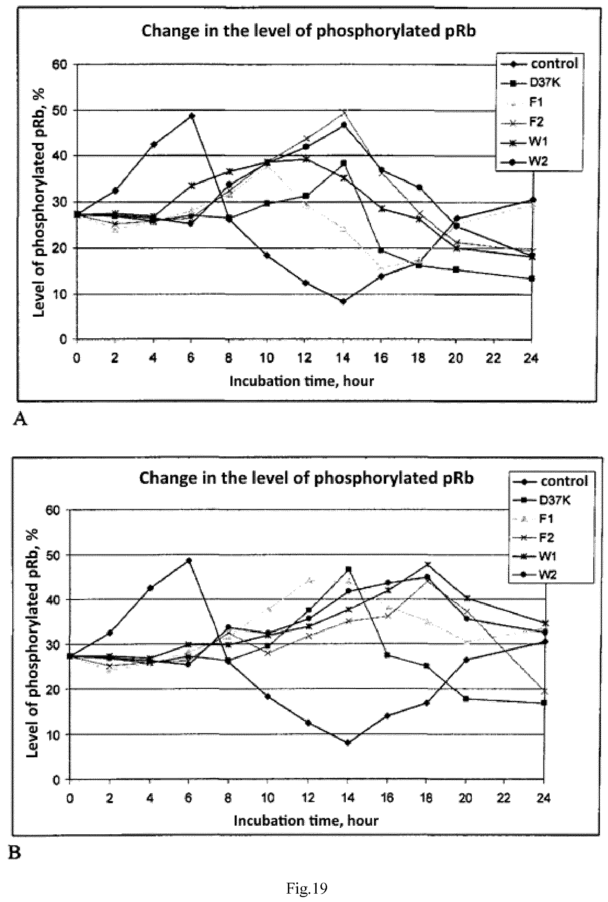

To determine the amount of "underphosphorylated" pRb, a series of experiments was performed, where the study peptide sequences were added to the cell cultures, synchronized with the help of depleted cell culture medium method (MCF-7, HCT-116), after removing the synchronization block, then the cells were sampled every two hours. A part of the cells from each plate was fixed to further define the phases of the cell cycle, and to determine the amount of the "underphosphorylated" pRb. Also a negative control was set in the form of synchronized cells samples, but without added peptide. It was shown that introduction of the peptide sequences into the cell culture inhibits the pRb phosphorylation and reduces its maximum level in the culture (Figure 19).

As it is seen in Figure 19, the level of "underphosphorylated" pRb begins to grow after removal of the synchronization block in the control samples without addition of the peptide, and to a certain point (4 or 6 hours of incubation), depending on the cell line, it reaches a maximum and then begins to decline. In the samples with peptides the level of "underphosphorylated" pRb begins to increase later and reaches its maximum after 12-18 hours of incubation, the delay in reaching the maximum level of pRb depends on concentration of the peptide sequences. For the culture of MCF-7 cells (Figure 19) the greatest delay in the formation of ""underphosphorylated" pRb sequence was achieved with F26K-2 and W26K-2. In the study of changes in the level of "underphosphorylated" pRb in the culture of HCT-116 cells when incubated with the peptide sequences, the greatest effect of the delay was obtained for the sequences F26K-1 and F26K-2. For the HCT-116 culture we performed 3 tests to determine the level of "underphosphorylated" pRb. Cells were synchronized by depleted medium (48 hours in medium containing 0.5% PBS), when changing the medium to medium containing 10% PBS the target sequences were added to the culture in concentrations of 10 and 40 µM. We analyzed the level of "underphosphorylated" pRb every two hours; the maximum incubation time was 24 hours.

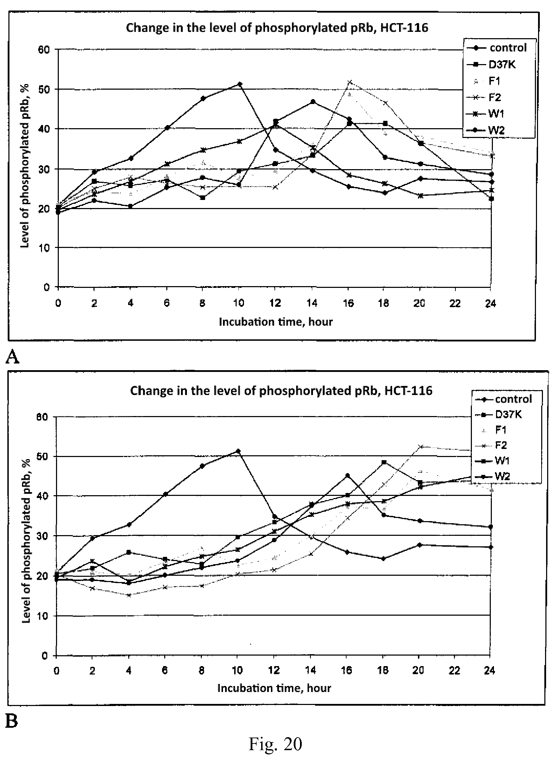

As shown in Figure 20, for the HCT-116 culture the largest effect from delay in formation of "underphosphorylated" pRb was exerted by sequences F26K-1 and F26K-2, as for these sequences the maximum level of "underphosphorylated" pRb, at peptide concentration of 10 µM, was observed after 16 hours of incubation, and at concentration of 40 µM after 20 hours. For the D37K sequence, the effect of the delay was observed to be the same regarding the time, but the level of "underphosphorylated" pRb was lower.

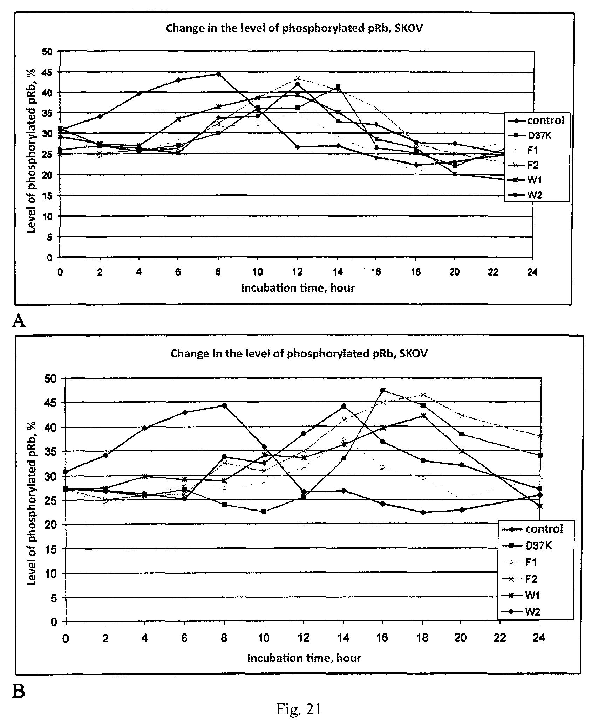

In the study of the "underphosphorylated" pRb level using SKOV cell line, the delay effect in formation of "underphosphorylated" pRb was lower than for cultures MCF-7 and HCT-116, although this cell line was previously shown to have the proliferation delay effect during incubation of the cells with the study peptide sequences. Figure 21 shows the change in the level of "underphosphorylated" pRb in SKOV cells culture with cell exposed to sequences D37K, F26K-1, 2-F26K, W26K-1, W26K-2 at concentrations of 10 µM (A) and 40 µM (B). Obvious "underphosphorylated" pRb formation delay effect compared to the control sample, however, in the case of SKOV cell line, was somewhat lower than that of cultures of MCF-7 and HCT-116. D37K sequence, in this case, has the strongest effect, but F26K-2 also causes the delay in formation of "underphosphorylated" pRb for 4-10 hours, as compared to the control.

Thus, it was found that the investigated peptide sequences D37K, F26K-1, 2-F26K, W26K-1 and W26K-2 are capable of inducing apoptosis in actively proliferating cells, and to inhibit proliferation processes. Among the investigated sequences the most pronounced cytotoxic and cytostatic effects were displayed by the sequences F26K-1 and F26K-2, during incubation with which the apoptosis has slightly higher values for proliferating cells, delay cell proliferation (increased numbers of cells in G0/G1 - phase of the cell cycle), concentration-depended reduced Bcl-2. Other investigated sequences also displayed cytotoxic and cytostatic effects, but those were less marked.

### Example 6. Study of the combined effect of internalized sequences and drugs

A series of experiments were set using HCT-116 cells culture to study the combined effects of internalized sequences F26K-1 and D37K and drugs with antitumour activity (Taxol, Etoposide). Given below are the averages of three experiments. Medications and internalized sequences were added to the culture medium at final concentrations of 1, 10 and 30 µM and were incubated for 24 hours. The cytotoxic effect was evaluated by MTT assay. This method is based on the ability of mitochondrial cytoplasmic dehydrogenases of metabolically active live cells to restore the non-stained forms of 3-4, 5-dimethylthiazol-2-yl-2, 5-diphenylterarazole (MTT reagent) to blue crystalline pharmazane soluble in dimethyl sulfoxide (DMSO).

To perform the MTT assay the cells at concentration of 2 x 10⁶/ml were placed in wells of a 96-well plate with medium volume of 100 µl, incubated for 24 hours, then the medium was replaced with the medium containing the drug agent (DA) and internalized sequence, incubated for 24 hours, the medium was sampled and 30 µL of the pure "complete" medium was added, and then PBS 170 µL and MTT 20 µL were added. Incubation was performed at 37°C for 30 min. The supernatant was collected.

DMSO (dimethyl sulfoxide) 200 µL were added and incubated for 15 min in the dark at room temperature. The values of the optical density were read on ELISA reader (Immunoassay Analyzer) at 492 nm.

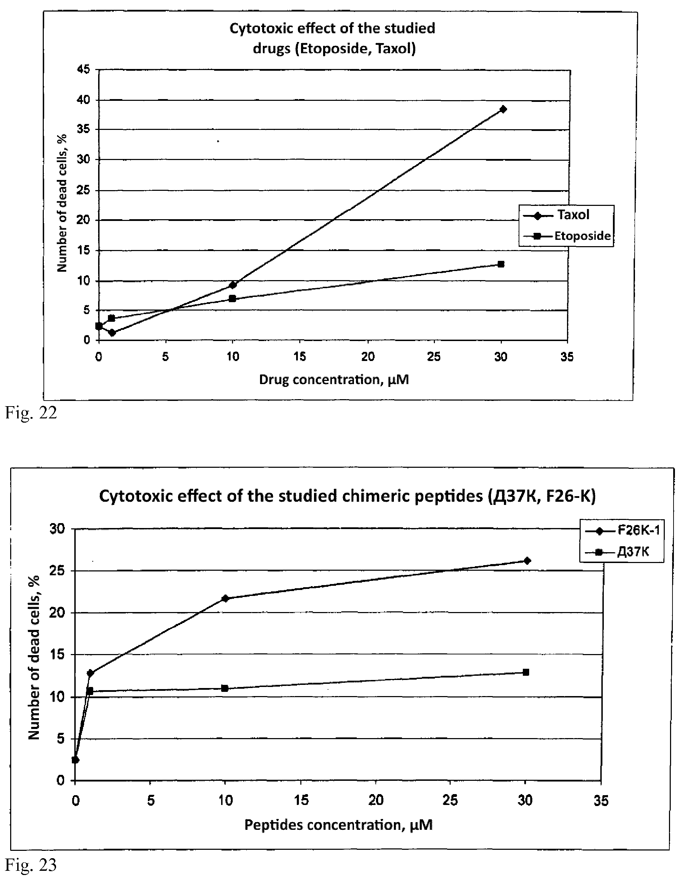

Figure 22 shows the change in the number of dead cells under the influence of drugs on cell culture. The cytotoxic effect of Etoposide in this range of concentrations and for a given cell line was minimal. Taxol produced a pronounced cytotoxic effect at concentrations of 30 µM. For the study sequences it was shown that the sequence F26K-1 induces cell death, and there was a dependence of the number of dead cells on the concentration of the sequence in the culture medium (Figure 23).

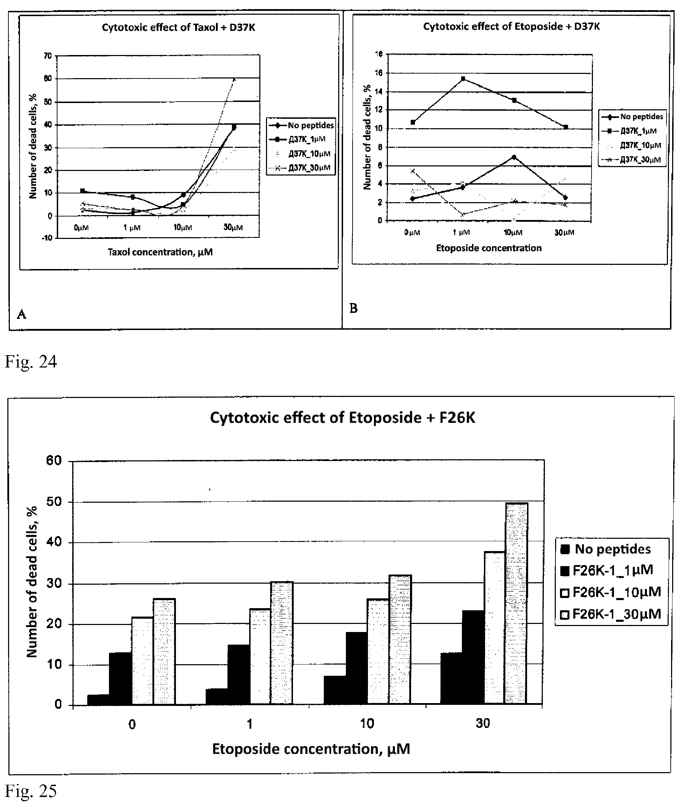

In the study of the combined effect of the sequence D37K and drugs, Taxol, Etoposide, the culture HCT-116 did not show the enhancement of cytotoxic effects of drugs (Figure 24). The number of dead cells was the same as when exposed to pure drugs.

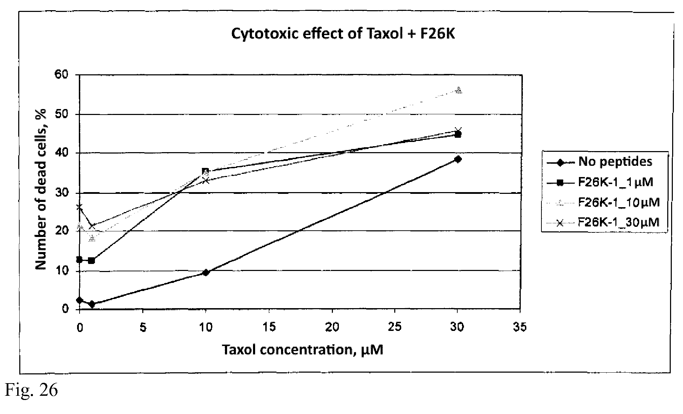

In the study of the combined effects of the sequence F26K-1 and drugs there was summation of the cytotoxic effects. The most clear summation effect was observed during co-incubation of cells with the sequence F26K-1 and Etoposide (Figure 25). During incubation of the cells with the sequence F26K-1 and Taxol the summation effect was observed at concentrations of 1 and 10 µM of F26K-1, but it was lost when the concentration of F26K-1 sequence increased to 30 µM (Figure 26). This effect was repeated in all experiments, and it was not possible to explain it at this stage, possibly a mechanism of oversaturation in response to the hyperactivation of the proapoptotic mechanisms, including pathways of feedback regulation, took place, and as a result, the inhibition of cell death occurred.

In cell culture MCF-7 (breast cancer) there was investigated the combined effect of DA, 5-fluorouracil and Taxol, and the sequences D37K and F26K-2 (in the cytotoxicity studies the sequence had the most pronounced effect on the MCF-7 culture). The cells were grown in 24-well plates to 70% of the monolayer, followed by replacement of the medium with the medium containing the target sequence and drugs in concentrations of 10, 20 and 30 µM. The incubation time was 24 hours. Analyzed cells, which entered into apoptosis, were detected using Annexin and propidium iodide staining, flow cytometry.

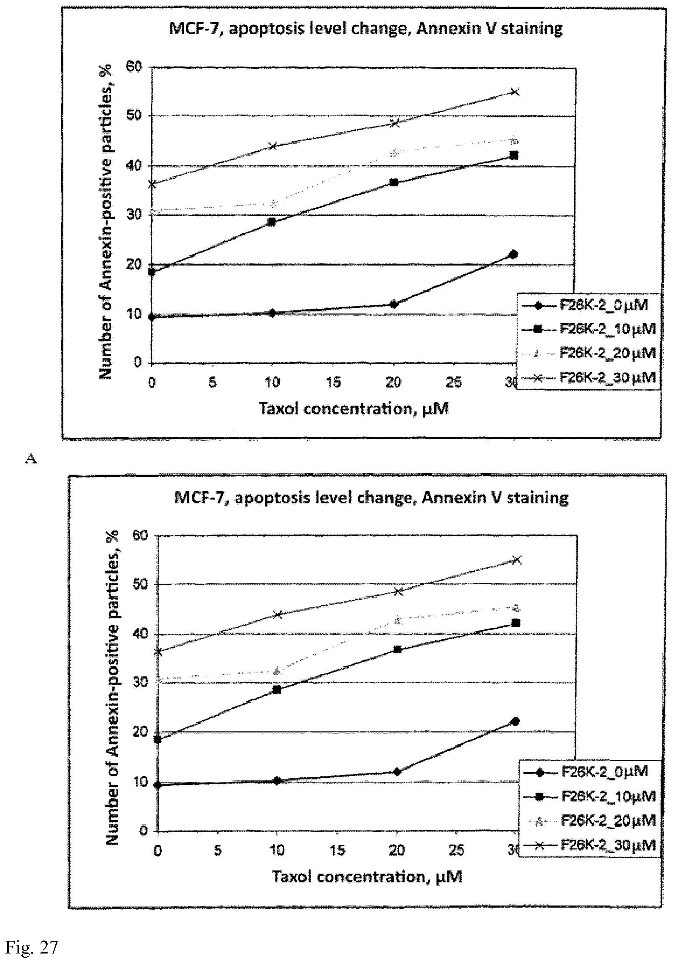

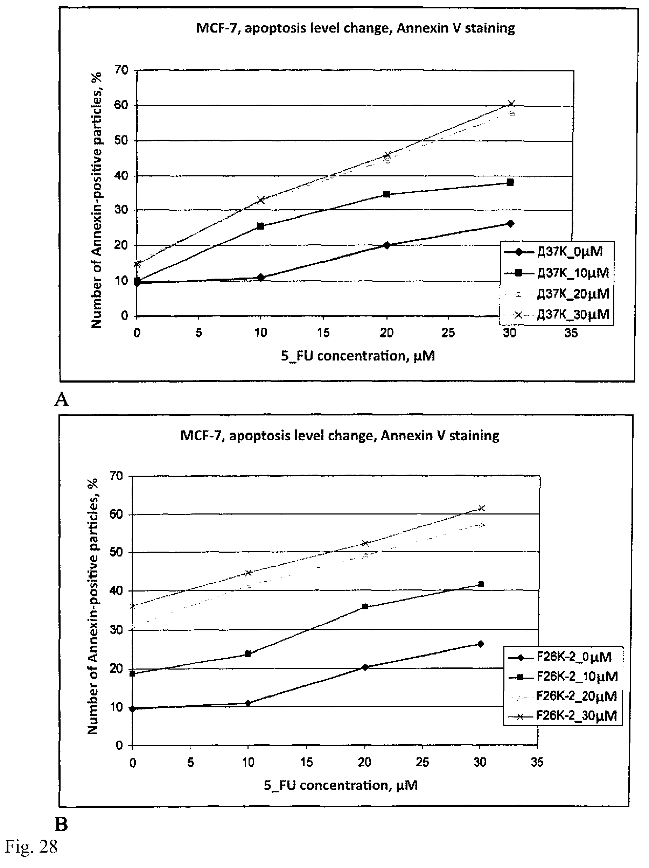

It was shown that the combined use of Taxol and F26K-2 sequence results in summation of cytotoxic effects, manifested as the increase in the level of apoptosis in proportion to the concentration of the drug. This effect is slightly reduced with increasing the concentration of F26K-2 sequence to 30 µM (Figure 27). In case of D37K sequence, at a concentration of 10 µM the cytotoxic effect is minimal, and the effects of the concentrations of 20 and 30 µM are virtually identical (Figure 27, A). In case of the combined use of 5-fluorouracil and internalized sequences D37K and F26K-2 a linear amplification was obtained for both investigated sequences, depending on the concentration of the cytotoxic effect. Moreover, with comparable values for the number of the apoptotic particles and reduced effect with increasing the concentration of peptide sequence to 30 µM (Figure 28).

Cell culture H460 (lung cancer) was used to investigate the effect of the combined sequences F26K-1, F26K-2, W26K-1, W26K-2, and Etoposide. In this test, the cells were incubated in 24-well plates to form 70% of the monolayer, and then the medium was replaced with the medium containing the internalized sequence at concentrations of 5, 10, 20 and 30 µM and Etoposide at concentration of 10 µM. The incubation time was 24 hours. The apoptosis was examined by Annexin-propidium iodide staining (early apoptosis) and the apoptosis level was determined using the number of particles with fragmented DNA, subdiploid peak was detected when staining fixed cells with propidium iodide.

It was shown that all investigated sequences possess apoptotic pronounced effect with respect to this cell culture, and the level of apoptosis was significantly increased with concomitant use of internalized peptide sequences and Etoposide.

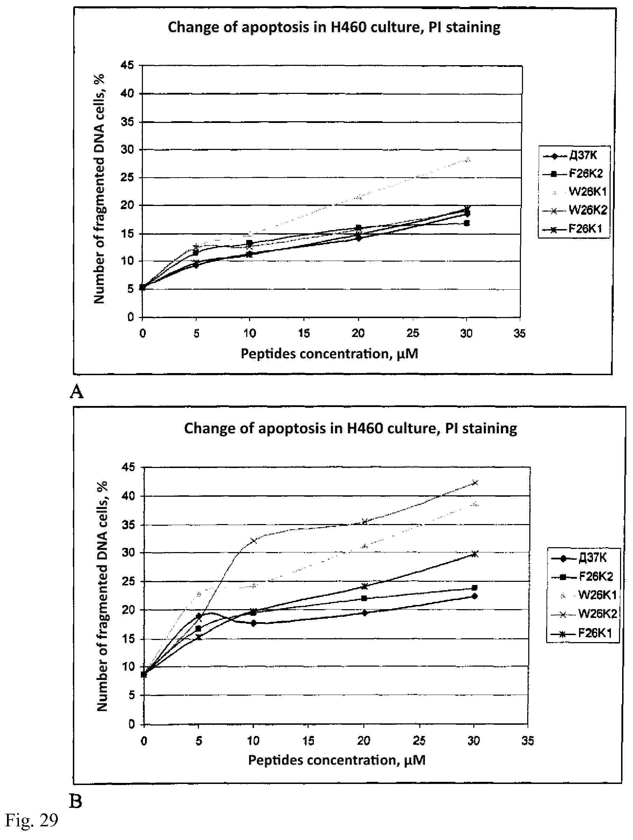

Figure 29A shows the changes in the level of apoptosis in the cells culture H460 upon incubation with the peptide sequences without Etoposide, incubation time 24 hours, the apoptosis was analyzed as a subdiploid peak. In Figure 29 there is an increase in the apoptosis level upon incubation of the cells with the sequences and Etoposide.

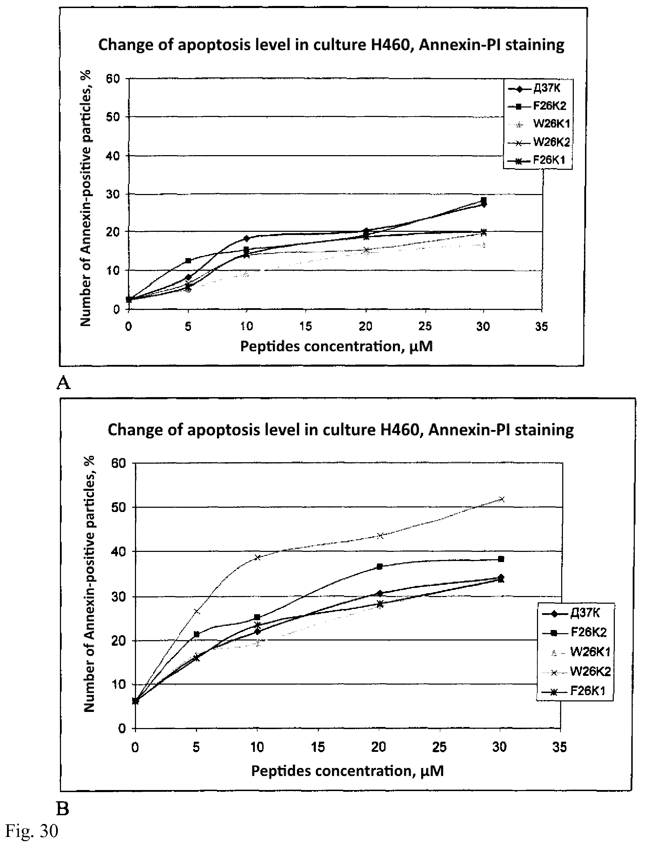

Figure 30 shows the same samples, but stained by dual labeled AnnexinV-PI, marked increase in early apoptotic cells positive for Annexin, when exposed to culture peptide sequences (A) and summation of the effects of the peptide sequences and Etoposide (B).

For the culture H460 the modified experiments were also set in order to study the combined effects of Etoposide and the peptide sequences. The cells were incubated in a 24-well plate to form 70% of the monolayer, and then the medium was replaced with the medium containing Etoposide at concentration of 10 µM, then the incubation was performed for 24 hours. The medium was replaced with the one containing internalized sequence at concentrations of 5, 10, 20 and 30 µM without Etoposide. Incubation was performed for 24 hours. The apoptosis was examined by Annexin-propidium iodide staining (early apoptosis), the apoptosis level was determined by the number of particles with fragmented DNA, and the subdiploid peak was determined by staining fixed cells with propidium iodide.

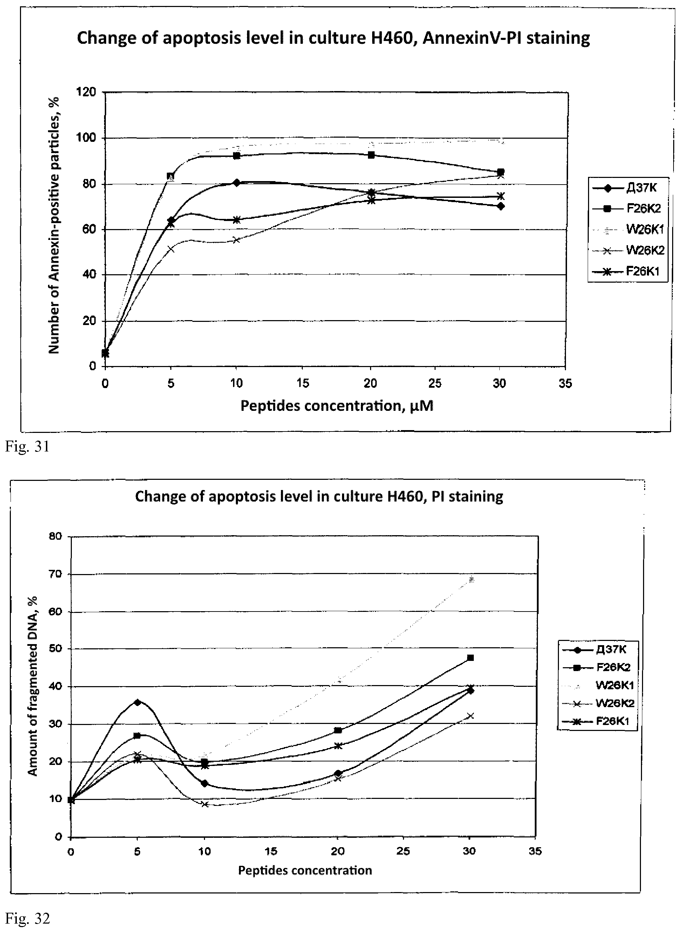

A sharp increase of the apoptosis at concentrations of the sequences F26K-1 and W26K-2 of 5 µM, was obtained, and it comprised over 80% (Figure 31).

The level of cells with fragmented DNA, which corresponds to later apoptotic processes, had a different dependence form, but dependence type was identical for all investigated peptide sequences (Figure 32). With this experiment design the overall increase of the apoptosis was observed compared to the experiments, in which the drugs were added to the culture medium simultaneously with the internalized sequences. This effect may be explained by the delay in proliferation, which is induced by Etoposide (blocking exit from the S-phase), while the internalized sequences, having the ability of cyclin-dependent kinases, stimulate the launch of apoptosis in the cells arrested by Etoposide.

Thus, in the course of the study it was shown that all studied peptide sequence (D37K, F26K-1, 2-F26K, W26K-1, W26K-2) had the ability to induce the apoptosis, affecting the cell proliferation processes. The level of effects depended on the sequence and on the cell culture. Comparing the peptide sequences it was showed that W26K-1 was the "weakest", and the remaining sequences displayed the cytotoxic effects superior to previously studied D37K. The opportunity to use the combination of internalized sequences and drugs, used in medical practice, is highlighted. The enhancement of cytotoxic properties of antitumour drugs is shown.

### Example 7. Study of the internalized peptides penetrating power

To register the penetration of peptides into the cell and to study the dynamics of accumulation we used identical chimeric sequence conjugated to a fluorescent label - flourestsein-isothiocyanate (FITC). Since including FITC molecules into the ready polypeptide product often results in a substantial change of its physical-chemical properties and loss of physiological activity, a lysine molecule, which carried a single FITC molecule, was attached at the N-terminus of the original sequence.

The light fluorescence microscopy showed that the studied peptides bind to cells and penetrate the cell lines Raji, Jurkatt, A549, 293 and human peripheral blood lymphocytes. Flow cytometry showed binding of the peptide to cells and no effect of fluorescence quenching with trypan blue, indicating the accumulation of the peptide inside the cell.

The most objective data on the accumulation of the peptide inside cells were obtained using the method of laser scanning microscopy. The protein labeled with fluorescein isothiocyanate was dissolved in 0.9% NaCl. The cell lines A549, 293 were grown on sterile slides and placed in a special chamber directly under the microscope eyepieces. The medium was replaced by the medium with the test protein. Saturation of the protein was observed over time. In addition, the layer by layer scanning of cells was performed to determine the peptide localization.

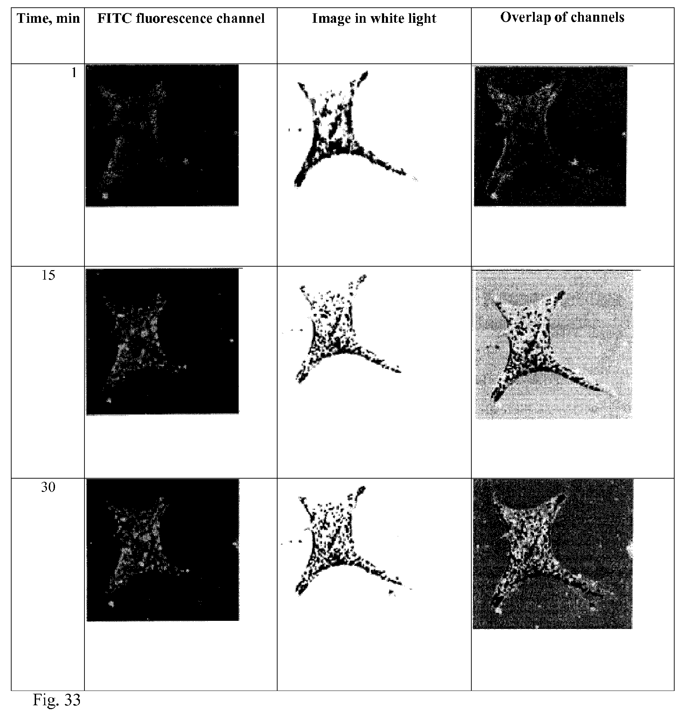

Analysis of the images at this enhancement did not reveal the location of the pre-emptive p16_pAntp protein in the cell. Obviously, the protein is distributed uniformly in the cell compartments. The penetration of the protein into the cells occurs quickly enough. Soon after 15 minutes of incubation its homogeneous distribution in the intracellular space can be observed (Fig. 33).

The rate of penetration of the peptide in human peripheral blood lymphocytes and B lymphocytes cell lines (Raji, Jurkatt) was evaluated using the method of flow cytometry. This method allows to explore a large concentration of cells in time and is convenient to estimate the rate of penetration of the test peptide. We measured the fluorescence of lymphocytes exposed to the protein p16-rAnpt-FITC at pH 7.5 and pH 6.0. The principle of the method is based on a smaller FITC fluorescence quantum yield in solutions with more acidic pH. Since the measurement is done sufficiently rapidly, the pH inside the cell cannot be changed. Lymphocytes were incubated with peptide for 1-15 minutes, then 20 times volumes of the phosphate buffer at pH 6.0 and pH 7.5 were immediately added thereto. At the same time we estimated difference in fluorescence intensity. Most measurements of fluorescence intensity were not significantly different after 15 minutes of incubation. Thus, it can be assumed that after 15 minutes of incubation the peptide completely penetrates the cell.

To study the rate of accumulation of peptide in the cell we used flow cytometry method. For this purpose the FITC-labeled peptide was injected directly into the measuring tube of the flow cytometer, making it possible to record the changes in the dynamics of cell fluorescence. We investigated the mononuclear fraction of blood leukocytes from healthy donors, isolated on a Ficoll gradient. After a short lag phase, a rapid accumulation of the peptide in the normal lymphocytes cells was seen. The final concentration was achieved by time ∼ 1 min.

A similar kinetics of the peptide accumulation was obtained for malignant lymphomas cells. The models investigated included Jurkat line (derived from lymphoblastic leukemia and having a T-cell phenotype) and Raji-produced from B-cell Burkitt lymphoma.

The peptide penetrates in the tumour cells very rapidly. Time to maximum concentration was less than 1 minute. The accumulation kinetics was studied at room temperature (t = 20°C). It should be emphasized that the penetration mechanism of the study class of peptides has not been known yet. However, it was shown that the accumulation occurs equally effective even at temperature of +5°C and was not associated with the energy consumption in the cell, not mediated by cellular receptors and did not use phago- and pinocytosis pathway. The authors also confirmed the accumulation of the synthesized peptide, containing the internalized component, at various temperatures (to +5°C).

Since the study peptides can cross the cellular membranes in both directions, it can be assumed that the peptides, accumulated in the cell, may leave it upon a decrease of the extracellular concentration. Investigation of the processes of peptides export from cells may be the subject of separate studies.

Thus, as a result of experiments, it was shown that the accumulation kinetics of the chimeric peptide, containing the internalized fragment and protein p16INK4a fragment, has a degree character with C = const1+const2*t²- type dependence. It was also shown that the dynamics of intracellular accumulation and distribution of the peptide in normal and tumour cells was not different in nature and rate of accumulation.

### Example 8. Investigation of the antitumour activity of the D37Kpeptide in vivo (topical

### administration)

The thymus-deprived mice (Nude) were used to study the antitumour activity of the internalized chimeric peptide D37K.

The mice were inoculated with tumour human cell culture lines A549 and HCT-116 in amount of about 1 million cells per mouse.

39 mice were inoculated with A549 cells, of which 20 mice formed the control group, that later received placebo. 10 mice formed 1 experimental group. After formation of a palpable tumour (4 days after the cells transplantation) they had the 0.1 mg of test peptide entered directly into the tumour. 9 mice were included in 2^{nd} experimental group, they also had 0.2 mg peptide injected into the tumour bed after formation of a tumour nodule on day 4 post transplantation. The peptide in the experimental groups was administered once per two days. The experiment lasted 24 days.

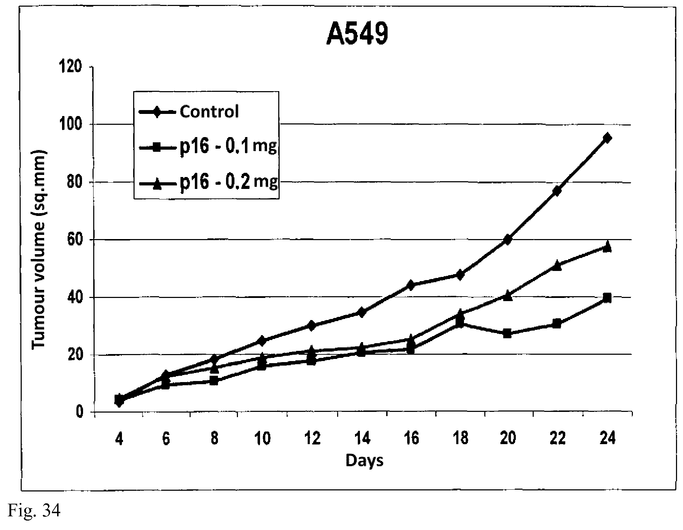

In the experimental groups we observed slowing of the tumour growth, and on day 6 after start of the peptide administration we were able to observe a marked decrease in the tumour volume in experimental groups compared with control (Fig. 34). The experiment lasted 24 days. During this time 10 injections of the chimeric peptide were performed in the experimental groups. In the control group on day 24 of the experiment an average tumour volume in mice was 95.24 mm³, one mouse died. In the first experimental group (dosage of the peptide - 0.1 mg) the average tumour volume was 39.29 mm³, one mouse died. In the second experimental group (dosage of the peptide - 0.2 mg) the average tumour volume was 57.51 mm³, but there were no dead animals.

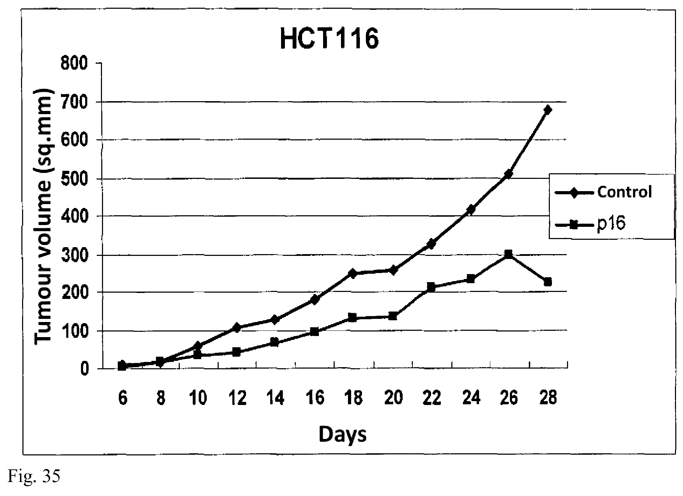

The animals were divided into two groups upon administering the transplanted culture of human HCT-116: control group - 10 mice not injected subsequently with analyzed peptide, and experimental group (10 mice), which had 0.1 mg of the analyzed P16_Antp chimeric peptide injected to the tumour nodule. The experiment lasted 28 days; 11 injections of the peptide were made in the experimental group; the first injection was made on day 6 after tumour cell inoculation. The tumour nodule from the HCT-116 cells was characterized by the significant sizes and ulceration. In the control group on day 28 of the experiment the average tumour volume reached 679 mm³, during the experiment three mice died. The experimental group had lower tumour growth rate (Fig. 35), on day 28 of the experiment the average tumour volume was 225.4 mm³, the number of dead animals in the experimental group comprised 2.

Fig. 36 presents several pairs of mice from different groups after 7 peptide injections. A significant decrease of the tumour nodule is observed in the experimental groups.

Topical administration of the chimeric peptide Antp_p16 results in significant (over 50%) inhibition of experimental models of human tumours growth (breast cancer, colorectal cancer).

### Example 9. Study of the cytotoxic properties of D37K in short-term cultures of human

### tumours

We investigated the antitumour activity of ***D37K***. 150 mg of ***D37K*** peptide were synthesized by solid-phase synthesis. We studied the effects on human tumours using the method of short-term cultures.

### Method of Tumour Short-Term Cultures Obtaining from Surgical Specimens.

Short-term cultures were obtained from surgical specimens. Sampling of section for preparing the culture was conducted at the earliest opportunity after surgery, and with the participation of the pathologist. The tissue piece was cut, the average size was 1.5 cm³. The tissue was ground mechanically; the cell suspension was placed in RPMI medium, containing 5% PBS. After 24 hours of incubation at 37°C and 5% CO₂, the medium was replaced with the medium containing the chimeric peptide (p16_Antp at concentration 40 µM) or, for a number of experiments, with Taxol in concentration of 100 nM or 500 nM, containing both peptide and Taxol. In the control samples the medium was replaced by fresh one. The zero point was established - 0 hours.

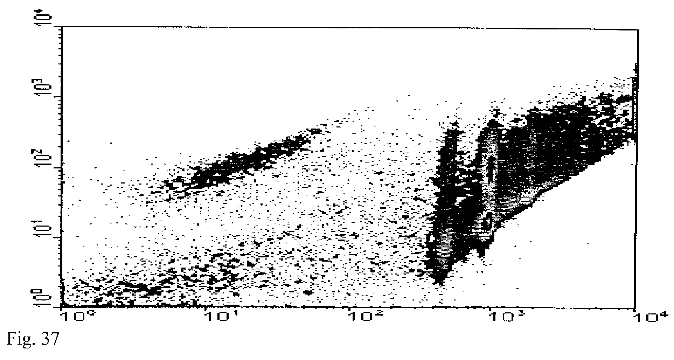

The incubation took place at 37°C and 5% CO₂ for 24 hours, for a series of experiments it was performed during 24 and 48 hours. The results were assessed with the help of flow cytometry method (for method description see "In vitro Methods") using samples dual AnnexinV-PI staining and fixed material PI staining. We assessed the level of particles positive according to AnnexinV (early apoptosis), the level of particles positive according to AnnexinV-PI double mark (late apoptosis), the number of particles in the non-fixed samples, able to include PI (necrosis); distribution of cells by cell cycle phases, subdiploid peak level (the number of particles with fragmented DNA, apoptosis). To detect the epithelial cells apoptosis we used a dual staining with antibodies to cytokeratin-FITC - PI. (Fig. 37).

In total we analyzed 126 tissue samples: 63 of them were the pathologic tissue samples (and 63 were the samples of normal tissues taken as controls). From 63 pathological specimens 47 tumours of malignant origin were investigated (10 - breast cancer, 15 - renal cancer, 6 - uterine cancer 4 - prostate cancer, 3 - ovarian cancer and lung cancer and 2 cases of stomach cancer, pancreatic cancer, bladder cancer). In addition, we studied fibroadenoma tissue samples (N = 9), and 7 samples of breast tissue with simple ductal hyperplasia.

The level of apoptosis was analyzed 24 and 48 hours after addition of the peptides at concentration of 40 µM. Some of the experiments were carried out to study the combined cytotoxic effect of traditional chemotherapy drugs and peptide *D37K* on the tumour cells.

### Example 10. Study of the antitumour activity of peptide D37K

Total results for cytotoxic activity of peptide D37K are shown in Table 3.

**Table 3. Mean level of the induced apoptosis in short-term tumour cultures exposed to peptide D37K. (24 hours, 40 µM).**

| Cancer type | Induced apoptosis level |
|---|---|
| Breast cancer | 25.8 |
| Renal cancer | 26.0 |
| Cervix cancer | 5.2 |
| Pancreatic cancer | 18.9 |
| Lung cancer | 13.5 |
| Stomach cancer | 35.4 |
| Prostate cancer | 15.4 |
| Bladder cancer | 24.7 |
| Ovarian cancer | 12.3 |

Analysis of the received results showed that the most sensitive forms of cancer for cytotoxic effect of peptide *D37K* are: renal cancer, breast cancer, stomach cancer, bladder cancer. It is worth to note that the short-term bladder cancer cultures are characterized by very high level of spontaneous apoptosis (to 60-70%). Despite the fact that addition of peptide only slightly increases this level, against this background the final results may be non-representative.

### Example 11. Study of the cytotoxic effect on the breast fibroadenoma cells

Interesting results were received when studying the breast fibroadenoma tissues samples. In short-term cultures of those cells with low level of spontaneous apoptosis (maximum to 20%, mean 12.8%) peptide p16 exerted significant cytotoxic effect and caused expressed apoptosis in 24 hours with the mean level of 38.2% with maximum to 80%. Less pronounced cytotoxic effect was seen for the breast cells with the signs of simple ductal hyperplasia (mean level of induced apoptosis 14.5%). The results agree with the author's data that studied the ratio of the proliferation activity and the spontaneous apoptosis level in pathological breast tissues. It was shown that the ratio of proliferation to spontaneous apoptosis is the highest for fibroadenoma tissue, with breast cancer cells on second place, followed by simple ductal hyperplasia and normal breast tissue. If one of the factors determining the sensitivity of cells to cytotoxic effect of peptides, containing proliferation inhibitors, then the fibroadenoma cells sensitivity to peptide D37K is rather logical. The detected effect of sensitivity of benign proliferative processes in the breast tissue may be prospective for treatment. Results of topical peptides injection for treating the transplanted solid tumours allow to presume its effectiveness when injected in the area of benign proliferative processes in the breast tissue. The acquired results allowed to detect a range of human tumours sensitive to peptide D37K. It was found that such localizations as breast cancer, colorectal cancer, stomach cancer, bladder cancer, renal cancer are the prospective objects for further study of cytotoxic (antitumour) effect of the studied peptide. The achieved results are novel since the available literature lacks the data on effect of the analyzed peptide on human tumours.

The specialist in the area will agree with interchangeability of some elements of the invention implementation variants. Similarly, some abovementioned elements and actions, as well as other known equivalents for each element, action or method can be combined with or replaced by one of common components, in accordance with the abovementioned principles, in order to form the compound. Despite the information was presented in the context of certain invention implementation variants and examples, the specialists in the area will agree, that it encompasses wider area of use than the use within the range of the abovementioned invention implementation variants, and/or can be used as the basis for multiple different modifications and equivalents. Thus the information is not limited by use only within the presented invention implementation variants.

## Claims

1. Chimeric peptide with antiproliferating activity, including the functional fragment and transport sequence, and the functional fragment includes the amino acid sequence, which is at least 60% similar to the amino acid sequence, chosen from the group SEQ ID NO: 1 - SEQ ID NO: 17.

2. Chimeric peptide with antiproliferating activity, including the functional fragment and transport sequence, and the functional fragment includes the amino acid sequence, chosen from the group SEQ ID NO: 1 - SEQ ID NO: 17.

3. Chimeric peptide according to claim 1, which is **characterized by** functional fragment, presented by the peptide, that includes the amino acid sequence SEQ IDNO: 1 or SEQ ID NO: 2, or SEQ ID NO: 3, or SEQ ID NO: 4.

4. Functional peptide with antiproliferating activity, including the amino acid sequence, which is at least 60% similar to the amino acid sequence, chosen from the group SEQ ID NO: 1 - SEQ IDNO: 17.

5. Functional peptide with antiproliferating activity, including the amino acid sequence, chosen from the group SEQ ID NO: 1 - SEQ ID NO: 17.

6. Chimeric peptide according to claim 1, which is **characterized by** the transport sequence from protein Antp or protein Tat.

7. Chimeric peptide according to claim 1 for treating a malignant tumour.

8. Chimeric peptide according to claim 1 for treating a benign tumour.

9. Functional peptide according to claim 4 for treating a malignant tumour.

10. Functional peptide according to claim 4 for treating a benign tumour.

11. Chimeric peptide according to claim 9, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

12. Functional peptide according to claim 9, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

13. The drug based on chimeric peptide according to claim 1 for treating a malignant tumour.

14. The drug based on chimeric peptide according to claim 1 for treating a benign tumour.

15. The drug according to claim 13, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

16. The drug based on the functional peptide according to claim 4 for treating a malignant tumour.

17. The drug based on the functional peptide according to claim 4 for treating a benign tumour.

18. The drug according to claim 16, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

19. The pharmaceutical composition with antiproliferating activity, which includes the chimeric peptide according to claim 1 or the functional peptide according to claim 4 as an active substance as well as pharmaceutically acceptable carriers.

20. The pharmaceutical composition according to claim 19, which includes additional pharmaceutically accepted active substance, chosen from the following group: alkylating agents, antimetabolites, plant alkaloids, antitumour antibiotics, platinum derivatives, camptothecin derivatives, altretamine, amsacrine, L-asparaginase, dacarbazine, estramustine, hydroxycarbamide, procarbazine, temozolomide, monoclonal antibodies, hormones, cytokines

21. The drug based on the pharmaceutical composition according to claim 19 for treating a malignant tumour.

22. The drug based on the pharmaceutical composition according to claim 19 for treating a benign tumour.

23. The drug according to claim 21, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

24. The drug based on the pharmaceutical composition according to claim 20 for treating a malignant tumour.

25. The drug based on the pharmaceutical composition according to claim 20 for treating a benign tumour.

26. The drug according to claim 24, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

27. Method of malignant tumour treatment, which includes an injection of chimeric peptide according to clause 1 or functional peptide according to claim 4 in a medically accepted dose to the mammal in need of such treatment.

28. Method of benign tumour treatment, which includes an injection of chimeric peptide according to clause 1 or functional peptide according to claim 4 in a medically accepted dose to the mammal in need of such treatment.

29. Method of treatment according to claim 27, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

30. Method of malignant tumour treatment, which includes an injection of the pharmaceutical composition according to claim 19 in a medically accepted dose to the mammal in need of such treatment.

31. Method of benign tumour treatment, which includes an injection of the pharmaceutical composition according to claim 19 in a medically accepted dose to the mammal in need of such treatment.

32. Method of treatment according to claim 30, which is **characterized by** a malignant tumour, chosen from the following group: colorectal cancer, renal cancer, lung cancer, breast cancer, bladder cancer, pancreatic cancer, uterine cancer, prostate cancer, stomach cancer, ovarian cancer, myeloma and malignant lymphoma.

33. Method of malignant tumour treatment, which includes an injection of the pharmaceutical composition according to claim 20 in a medically accepted dose to the mammal in need of such treatment.

34. Method of benign tumour treatment, which includes an injection of the pharmaceutical composition according to claim 20 in a medically accepted dose to the mammal in need of such treatment.

35. Method of treatment according to claim 33, which is **characterized by** a malignant tumour, chosen from the following group: colorectal cancer, renal cancer, lung cancer, breast cancer, bladder cancer, pancreas cancer, uterine cancer, prostate cancer, stomach cancer, ovarian cancer, myeloma and malignant lymphoma.


SEQUENCE LISTING

<110> Obshestvo S Ogranichennoy Otvetstvennostiu "METAMAX"

<120> Chimeric Peptid and Pharmaceutical Composition for Treating
Hyperproliferative Diseases

<130> 103919PCEP

<140> EP14778337.7

<150> EA201300313
<151> 03-04-2013

<160> 18

<210> 1
<211> 8
<212> PRT
<213> Homo sapiens

<220>
<223> functional sequence of protein inhibitor of cyclin-kinase

<400> 1
Phe Leu Asp Ala Leu Val Val Leu
1               5

<210> 2
<211> 8
<212> PRT
<213> Homo sapiens

<220>
<223> functional sequence of protein inhibitor of cyclin-kinase

<400> 2
Phe Leu Asp Ala Ile Leu Leu Ile
1               5

<210> 3
<211> 8
<212> PRT
<213> Homo sapiens

<220>
<223> functional sequence of protein inhibitor of cyclin-kinase

<400> 3
Trp Ile Thr Ala Leu Val Val Leu
1               5

<210> 4
<211> 8
<212> PRT
<213> Homo sapiens

<220>
<223> functional sequence of protein inhibitor of cyclin-kinase

<400> 4
TrpIleThrAlaIleLeuLeuIle
1               5

<210> 5
<211> 8
<212> PRT
<213> Homo sapiens

<220>
<223> functional sequence of protein inhibitor of cyclin-kinase

<400> 5
Trp Leu Asp Ala Leu Val Val Leu
1               5

<210> 6
<211> 8
<212> PRT
<213> Homo sapiens

<220>
<223> functional sequence of protein inhibitor of cyclin-kinase

<400> 6
Phe Val Asp Ala Leu Val Val Leu
1               5

<210> 7
<211> 8
<212> PRT
<213> Homo sapiens

<220>
<223> functional sequence of protein inhibitor of cyclin-kinase

<400> 7
Trp Ile Asp Ala Leu Val Val Leu
1               5

<210> 8
<211> 8
<212> PRT
<213> Homo sapiens

<220>
<223> functional sequence of protein inhibitor of cyclin-kinase

<400> 8
Phe Leu Thr Ala Leu Val Val Leu
1               5

<210> 9
<211> 8
<212> PRT
<213> Homo sapiens

<220>

<223> functional sequence of protein inhibitor of cyclin-kinase

<400> 9
Trp Ala Asp Ala Leu Val Val Leu
1               5

<210> 10
<211> 8
<212> PRT
<213> Homo sapiens

<220>
<223> functional sequence of protein inhibitor of cyclin-kinase

<400> 10
Trp Ile Thr Ala Val Val Ala Val
1               5

<210> 11
<211> 8
<212> PRT
<213> Homo sapiens

<220>
<223> functional sequence of protein inhibitor of cyclin-kinase

<400> 11
Trp Ile Thr Ala Leu Ala Ala Leu
1               5

<210> 12
<211> 8
<212> PRT
<213> Homo sapiens

<220>
<223> functional sequence of protein inhibitor of cyclin-kinase

<400> 12
Trp Ile Thr Ala Ile Ala Ala Ile
1               5

<210> 13
<211> 8
<212> PRT
<213> Homo sapiens

<220>
<223> functional sequence of protein inhibitor of cyclin-kinase

<400> 13
Trp Ile Thr Ala Leu Leu Ile Ile
1               5

<210> 14
<211> 8
<212> PRT
<213> Homo sapiens

<220>
<223> functional sequence of protein inhibitor of cyclin-kinase

<400> 14
Phe Leu Asp Ala Leu Leu Val Leu
1               5

<210> 15
<211> 8
<212> PRT
<213> Homo sapiens

<220>
<223> functional sequence of protein inhibitor of cyclin-kinase

<400> 15
Phe Leu Asp Ala Leu Val Leu Leu
1               5

<210> 16
<211> 8
<212> PRT
<213> Homo sapiens

<220>
<223> functional sequence of protein inhibitor of cyclin-kinase

<400> 16
Trp Val Asp Ala Leu Val Val Leu
1               5

<210> 17
<211> 8
<212> PRT
<213> Homo sapiens

<220>
<223> functional sequence of protein inhibitor of cyclin-kinase

<400> 17
Trp Ile Thr Ala Leu Ile Ile Leu
1               5

<210> 18
<211> 37
<212> PRT
<213> Homo sapiens

<220>
<223> functional sequence of protein inhibitor of cyclin-kinase

<400> 18
Asp Ala Ala Arg Glu Gly Phe Leu Asp Thr Leu Val Val Leu His Arg
1               5                   10                  15
Ala Gly Ala Arg  Ser Arg Gln Ile Lys Ile Trp Phe Gln Asn Arg Arg
            20                   25                  30
Met Lys Trp Lys Lys
        35